# EUROPEAN PATENT APPLICATION

(11) **EP 4 722 351 A1**
(43) Date of publication of application: **08.04.2026**
(21) Application number: 24815509.5
(22) Date of filing: 29.05.2024
(51) Int. Cl.: C12N 7/00, A01N 63/40, A01P 3/00, C12N 1/00, C12N 1/02, C12N 1/06, C12N 1/20, C12N 15/11, C12N 15/34, C12Q 1/04

(54) **BACTERIOPHAGE HAVING BACTERIOLYTIC ACTIVITY AGAINST XANTHOMONAS BACTERIA**

(30) Priority: 31.05.2023 JP 2023089657; 31.05.2023 JP 2023089686; 31.05.2023 JP 2023089379
(71) Applicant: Kaneka Corporation, Osaka-shi, Osaka 530-8288 (JP); Rakuno Gakuen University, Ebetsu-shi, Hokkaido 069-8501 (JP); Mitsui & Co., Ltd., Chiyoda-Ku Tokyo 100-8631 (JP)
(72) Inventor: YOSHIDA Shinichi, Iwata-shi, Shizuoka 438-0802 (JP); DOJUN Nobuhiko, Iwata-shi, Shizuoka 438-0802 (JP); IWANO Hidetomo, Ebetsu-shi, Hokkaido 069-8501 (JP); KUDO Hitoshi, Tokyo 100-8631 (JP)
(74) Representative: Hoffmann Eitle
(86) International application number: PCT/JP2024/019668
(87) International publication number: WO 2024/248028

(57) **Abstract**

To control plant diseases caused by Xanthomonas bacteria, a novel bacteriophage exhibiting bacteriolytic activity specifically against Xanthomonas bacteria was isolated, and a plant disease control composition containing the bacteriophage as an active ingredient has been developed and provided. Provided are a bacteriolytic agent comprising a bacteriophage that has a novel genomic DNA sequence and exhibits bacteriolytic activity specifically against Xanthomonas bacteria, and a plant disease control composition containing the bacteriophage as an active ingredient.

## Description

### Technical Field

The present invention relates to a bacteriolytic agent composed of a bacteriophage, a composition for plant disease control comprising the same, and a method for controlling plant disease.

### Background Art

A bacteriophage (herein often abbreviated simply as a "phage") is a generic term for viruses that infect only bacteria. Many phages, also called bacteriolytic phages, are specifically attached to target bacteria (host) as a host, then inject their own DNA, and are self-amplified utilizing the translational mechanism of the bacteria. Furthermore, the bacteria are bacteriolyzed, and consequently, the amplified phages are diffused, and an infection into new target bacteria is repeated (Non-Patent Literature 1).

Many of the bacteria of the *Xanthomonas* genus are known to be pathogenic bacteria for diseases of plants, for example, various crops. In common customary control, a copper fungicide or an antibiotic has been used. However, there are many problems, such as a drug effect, drug poisoning, and as well as possible causes for the disruption of a bacterial lawn balance. Hence, recent interest has focused on a method using a phage as a new control method (Non-Patent Literature 2).

An example of a phage that exhibits bacteriolytic ability to bacteria of the *Xanthomonas* genus is reported, for example, in Patent Literature 1 to 3 and Non-Patent Literature 2. However, the host range of the phages is extremely narrow. Hence, it is still important to search for a new phage and to find a phage having higher bacteriolytic ability.

### Citation List

### Patent Literature

Patent Literature 1: JP2016-32435A
Patent Literature 2: JP2021-102635A
Patent Literature 3: WO2017/113029

### Non-Patent Literature

Non-Patent Literature 1: Sharma S. et al., Folia Microbiol., 2017, 62:17-55
Non-Patent Literature 2: Nakayinga R. et al., BMC Microbiology, 2021, 21:291

### Summary of Invention

### Technical Problem

To solve the above-described problems, the present inventors have aimed their attention at a bacteriophage. Differently from a conventional copper fungicide or antibiotic, a phage, which is a virus, is a natural product, and the manifestation of its drug poisoning has hitherto not been reported. Additionally, a phage has a very high specificity to a host, and thus only targets bacteria of a specific genus or species, having an extremely limited influence on a bacterial lawn balance. Additionally, a phage is harmless not only to an animal such as a human but also a plant, and is very safe. Accordingly, to inhibit damage causable to crops by a plant disease caused by bacteria of the *Xanthomonas* genus, a novel phage that exhibits bacteriolytic ability against bacteria of the *Xanthomonas* genus is isolated. The further object is to provide a composition comprising the phage as an active component, and to use the composition for disease control, the detection of pathogenic bacteria, and the like.

### Solution to Problem

The present inventors have isolated a novel phage from natural dirty water and soil, using a method for detecting a bacteriolytic plaque formed on a soft agar medium having bacteria of the *Xanthomonas* genus cultured thereon, and have analyzed the genomic sequence of the phage. Consequently, the phage has been revealed as having a novel genomic DNA sequence, the analogous genomic sequence of which is not known at all. The present invention has been completed on the basis of the above-described results of research and development. Specifically, the present invention provides the following.

[1-A] A bacteriolytic agent comprising a bacteriophage having a genomic DNA sequence comprising the nucleotide sequence of any of the following (1a) to (1c): (1a) the nucleotide sequence of SEQ ID NO: 1; (1b) the nucleotide sequence having addition, deletion, and/or substitution of one or a plurality of nucleotides in the nucleotide sequence of SEQ ID NO: 1; or (1c) a nucleotide sequence having a sequence identity of 95% or more to the nucleotide sequence of SEQ ID NO: 1.
[2-A] The bacteriolytic agent according to [1-A], wherein the bacteriolytic agent exhibits bacteriolytic ability against bacteria of the *Xanthomonas* genus.
[3-A] The bacteriolytic agent according to [2-A], wherein the bacteria of the *Xanthomonas* genus are at least one kind of bacteria selected from the group consisting of *Xanthomonas arboricola, Xanthomonas campestris,* and *Xanthomonas citri.*
[1-B] A bacteriolytic agent comprising a bacteriophage having a genomic DNA sequence comprising the nucleotide sequence of any one of the following (2a) to (2c) with a full length of 40,000 or less nucleotides: (2a) the nucleotide sequence of SEQ ID NO: 2; (2b) the nucleotide sequence having addition, deletion, and/or substitution of one or a plurality of nucleotides in the nucleotide sequence of SEQ ID NO: 2; or (2c) a nucleotide sequence having a sequence identity of 90% or more to the nucleotide sequence of SEQ ID NO: 2.
[2-B] The bacteriolytic agent according to [1-B], wherein the bacteriolytic agent exhibits bacteriolytic ability against bacteria of the *Xanthomonas* genus.
[3-B] The bacteriolytic agent according to [2-B], wherein the bacteria of the *Xanthomonas* genus *are Xanthomonas arboricola and*/*or Xanthomonas campestris.*
[1-C] A bacteriolytic agent comprising a bacteriophage having a genomic DNA sequence comprising the nucleotide sequence of any one of the following (3a) to (3c): (3a) the nucleotide sequence of SEQ ID NO: 3; (3b) the nucleotide sequence having addition, deletion, and/or substitution of one or a plurality of nucleotides in the nucleotide sequence of SEQ ID NO: 3; or (3c) a nucleotide sequence having a sequence identity of 90% or more to the nucleotide sequence of SEQ ID NO: 3.
[2-C] The bacteriolytic agent according to [1-C], wherein the bacteriolytic agent exhibits bacteriolytic ability against bacteria of the *Xanthomonas* genus.
[3-C] The bacteriolytic agent according to [2-C], wherein the bacteria of the *Xanthomonas* genus *are Xanthomonas arboricola and*/*or Xanthomonas campestris.*
[4] A composition comprising the bacteriolytic agents according to any of [1-A] to [3-C] as an active component(s).
[5] A composition for plant disease control, comprising the composition according to [4].
[6] The composition for plant disease control according to [5], wherein the plant disease is a plant disease caused by bacteria of the *Xanthomonas* genus.
[7] The composition for plant disease control according to [5] or [6], comprising another bacteriophage having bacteriolytic ability against the bacteria of the *Xanthomonas* genus.
[8] A method for controlling plant disease, comprising a contacting step of contacting the composition for plant disease control according to any of [5] to [7] with a target plant.
[9] A method for identifying bacteria of the *Xanthomonas* genus, comprising: a culturing step of culturing subject bacteria isolated from a plant tissue affected by a plant disease, to obtain a culture preparation; a mixing step of mixing the culture preparation and the bacteriolytic agent according to [2] or [3], to obtain a mixture; a mixture culturing step of culturing the mixture under predetermined conditions; and a determining step of determining that the subject bacteria are bacteria of the *Xanthomonas* genus, when the subject bacteria are bacteriolyzed after the mixture culturing step.
[10] The method according to [9], wherein, in the mixture culturing step, the mixture further comprises a soft agar containing liquid medium, and wherein the mixture is cultured on a solid medium.
[11] The method according to [9], wherein, in the culturing step, the culture preparation comprises a soft agar containing liquid medium, and wherein the culture preparation is cultured on a solid medium.
[12] The method according to any of [9] to [11], further comprising, before the culturing step, an isolating step of isolating subject bacteria from a plant tissue affected by a plant disease.

The present specification encompasses the disclosure of Japanese Patent Application Nos. 2023-089657, 2023-089686, and 2023-089379 that serve as a basis for the priority of the present application.

### Advantageous Effects of Invention

A bacteriolytic agent of the present invention and a composition comprising the same as an active component can bacteriolyze specific target bacteria.

A composition for plant disease control according to the present invention can prevent and inhibit a disease caused by specific target bacteria.

### Brief Description of Drawing

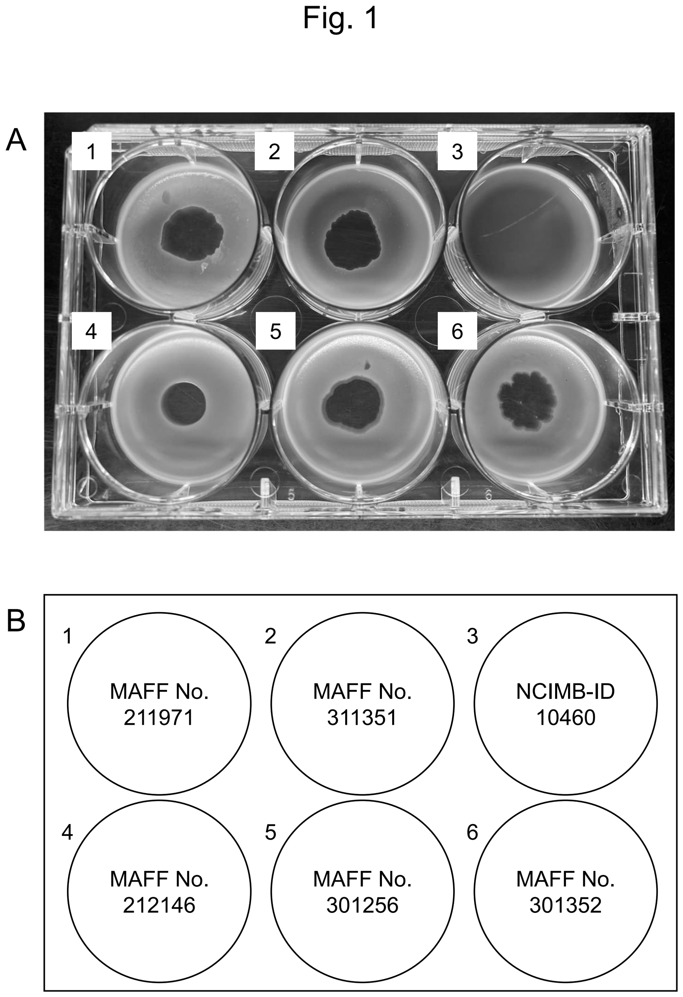

[Figure 1] Figure 1 shows the bacteriolytic ability of the 1st bacteriophage obtained in Example 1. Figure 1A shows a static culture with agar plates on which the bacteria of the *Xanthomonas* genus listed in Table 2 and *Pseudomonas fluorescens* as a control were spread and cultured, and onto the central portion of which a refined solution of the 1st phage was then dropped. Figure 1B is a diagram of the plates corresponding to Figure 1A, showing the bacteria listed in Table 2, which were spread on the respective plates.

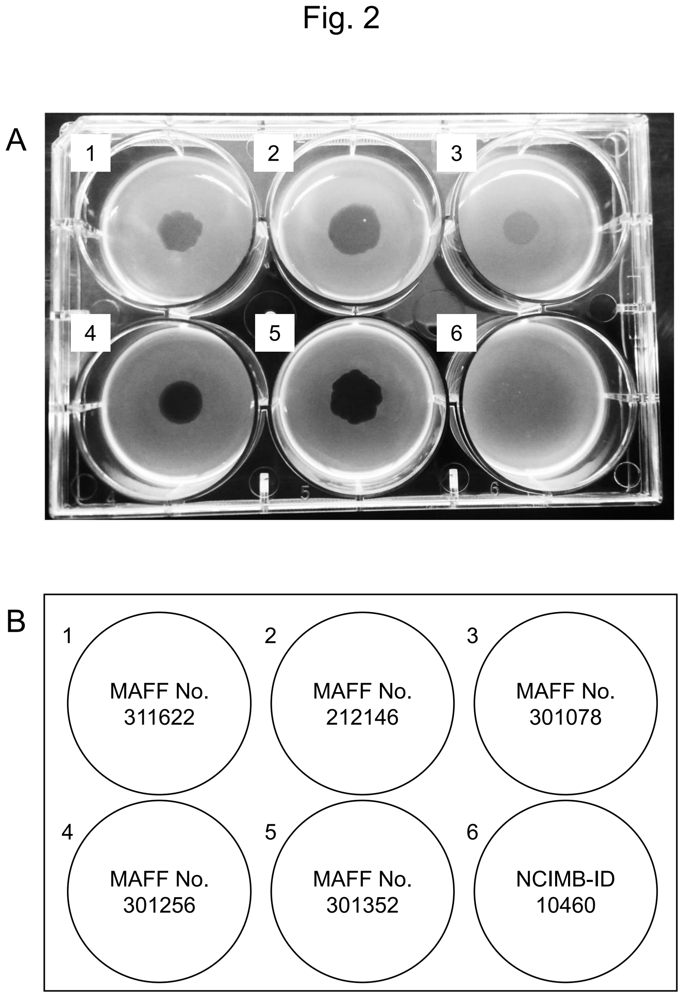

[Figure 2] Figure 2 shows the bacteriolytic ability of the 1st bacteriophage obtained in Example 1. Figure 2A shows a static culture with agar plates on which the bacteria of the *Xanthomonas* genus and *Pseudomonas fluorescens* as a control were spread and cultured, and onto the central portion of which a refined solution of the 1st phage was then dropped. Figure 2B is a diagram of the plates corresponding to Figure 2A, showing the bacteria listed in Table 2, which were spread on the respective plates.

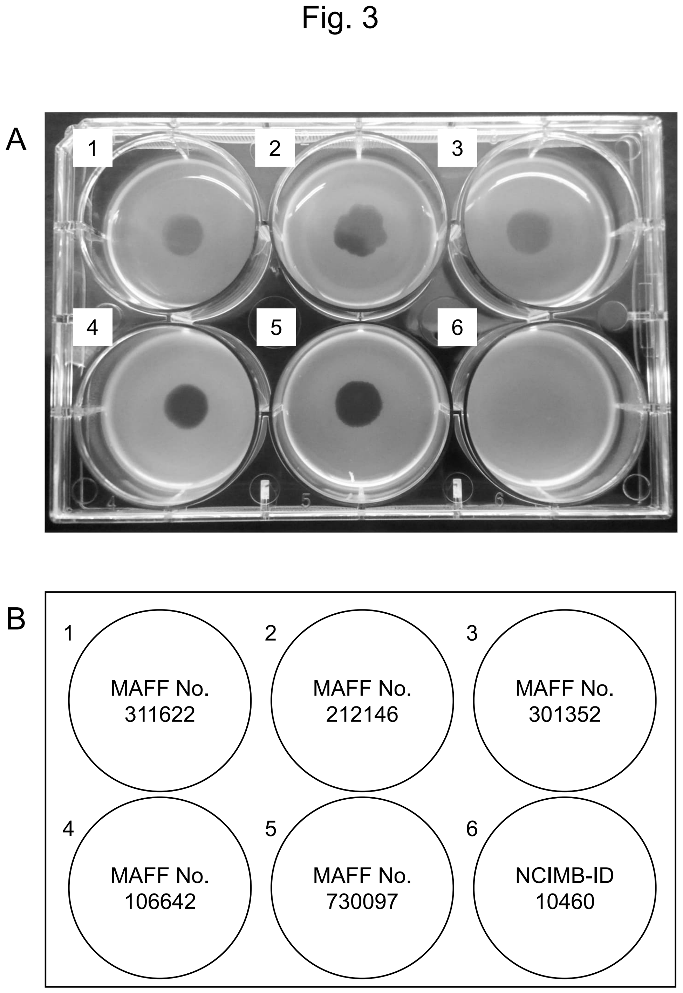

[Figure 3] Figure 3 shows the bacteriolytic ability of the 2nd bacteriophage obtained in Example 2. Figure 2A shows a static culture with agar plates on which the bacteria of the *Xanthomonas* genus listed in Table 4 and *Pseudomonas fluorescens* as a control were spread and cultured, and onto the central portion of which a refined solution of the 2nd phage was then dropped. Figure 2B is a diagram of the plates corresponding to Figure 2A, showing the bacteria listed in Table 4, which were spread on the respective plates.

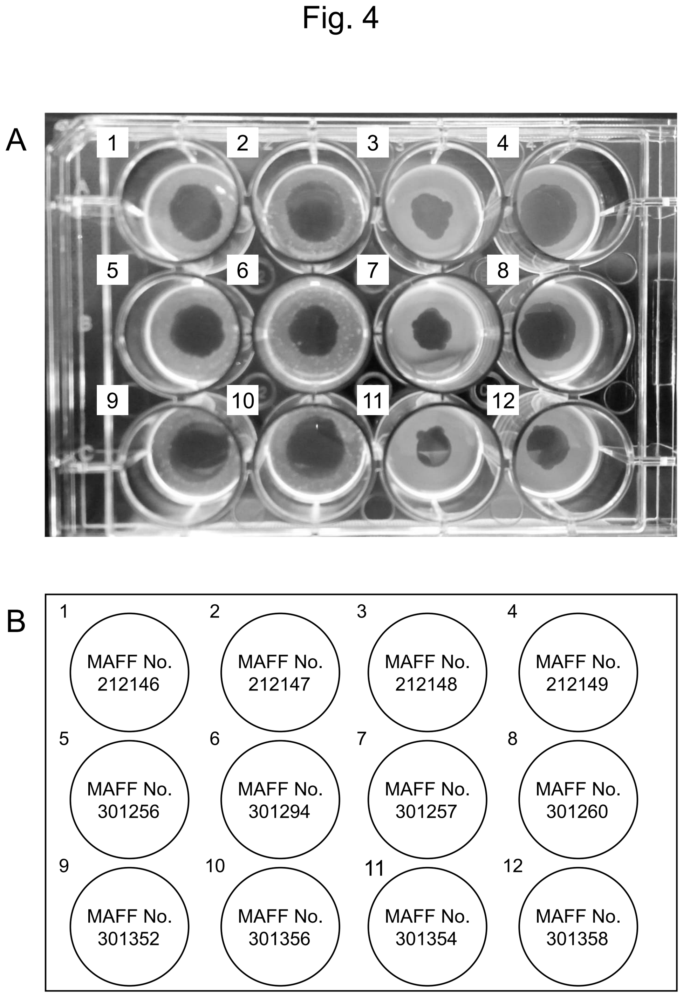

[Figure 4] Figure 4 shows the bacteriolytic ability of the 2nd bacteriophage obtained in Example 2. Figure 4A shows a static culture with agar plates on which the bacteria of the *Xanthomonas* genus listed in Table 4 and *Pseudomonas fluorescens* as a control were spread and cultured, and onto the central portion of which a refined solution of the 2nd phage was then dropped. Figure 4B is a diagram of the plates corresponding to Figure 4A, showing the bacteria listed in Table 4, which were spread on the respective plates.

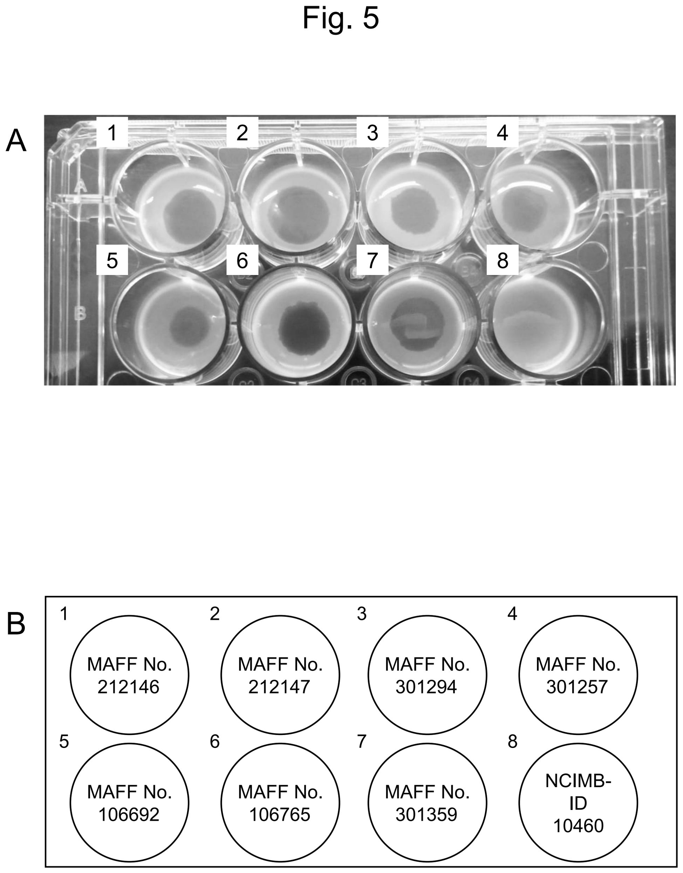

[Figure 5] Figure 5 shows the bacteriolytic ability of the 3rd bacteriophage obtained in Example 3. Figure 5A shows a static culture with agar plates on which the bacteria of the *Xanthomonas* genus and *Pseudomonas fluorescens* as a control, which are shown in Table 5, were spread and cultured, and onto the central portion of which a refined solution of the 3rd phage was then dropped. Figure 5B is a diagram of the plates corresponding to Figure 5A, showing the bacteria listed in Table 5, which were spread on the respective plates.

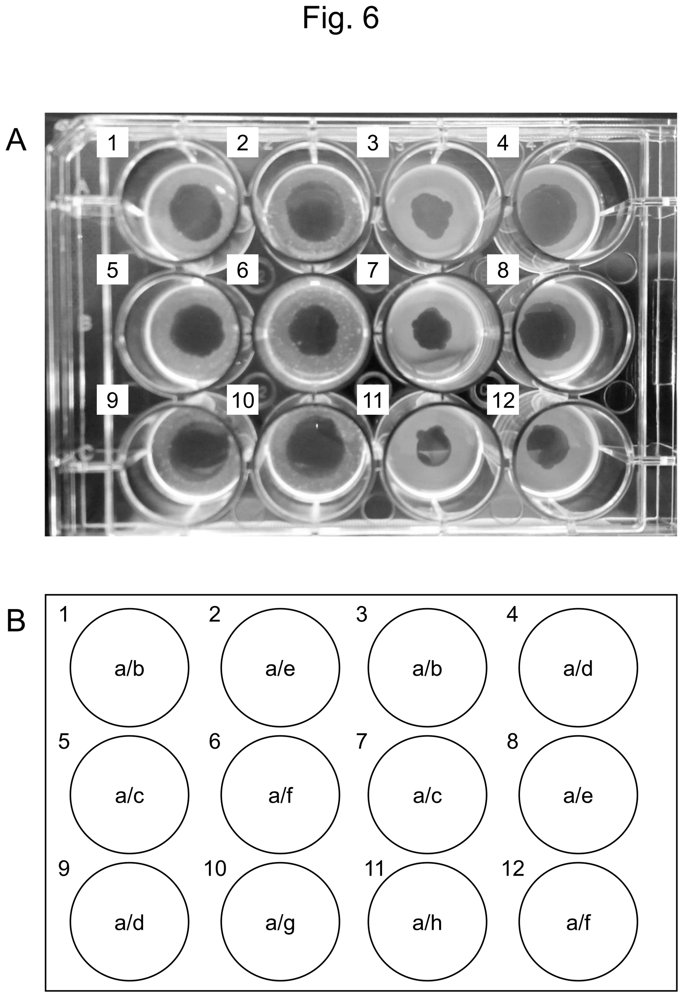

[Figure 6] Figure 6 shows the bacteriolytic ability of the combinations of the 1st bacteriophage and another bacteriophage tested in Example 4. Figure 6A shows a static culture on an agar plate on which the bacteria of the *Xanthomonas* genus were spread and cultured, and onto which a refined solution of phage was then dropped. In Figures 6A and 6B, the left two columns (1, 2, 5, 6, 9 and 10) each indicate a plate on which the bacteria with the ID MAFF No. 673005 was spread, and the right two columns (3, 4, 7, 8, 11 and 12) each indicate a plate on which the bacteria with the ID MAFF No. 301352 was spread. In Figure 6B, a indicates the 1st phage having the genomic DNA sequence of SEQ ID NO: 1, b indicates a phage having the genomic DNA sequence of SEQ ID NO: 4, c indicates a phage having the genomic DNA sequence of SEQ ID NO: 5, d indicates a phage having the genomic DNA sequence of SEQ ID NO: 6, e indicates a phage having the genomic DNA sequence of SEQ ID NO: 7, f indicates the 2nd phage having the genomic DNA sequence of SEQ ID NO: 2, g indicates the 3rd phage having the genomic DNA sequence of SEQ ID NO: 3, and h indicates a phage having the genomic DNA sequence of SEQ ID NO: 8. In the diagram, the mark "/" indicates that the phages before and after the mark are used in combination. For example, "a/f" indicates that the 1st phage and the 2nd phage are used in combination.

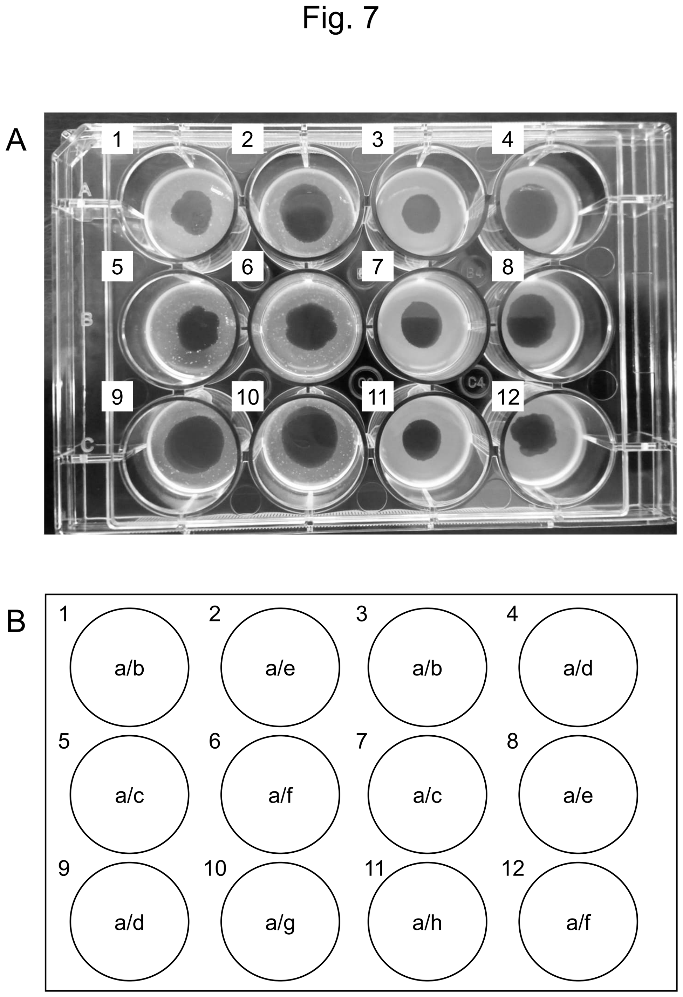

[Figure 7] Figure 7 shows the bacteriolytic ability of the combinations of the 2nd bacteriophage and another bacteriophage tested in Example 5. Figure 7A shows a static culture on an agar plate on which the bacteria of the *Xanthomonas* genus were spread and cultured, and onto which a refined solution of phage was then dropped. Figure 7B is a diagram of the plates corresponding to Figure 7A, showing the kinds of phages comprised in the refined solutions of phages dropped onto the respective plates. In Figures 7A and 7B, the left two columns (1, 2, 5, 6, 9 and 10) each indicate a plate on which the bacteria with the ID MAFF No. 673005 was spread, and the right two columns (3, 4, 7, 8, 11 and 12) each indicate a plate on which the bacteria with the ID MAFF No. 301352 was spread. In Figure 7B, a indicates the 2nd phage having the genomic DNA sequence of SEQ ID NO: 2, b indicates a phage having the genomic DNA sequence of SEQ ID NO: 4, c indicates a phage having the genomic DNA sequence of SEQ ID NO: 5, d indicates a phage having the genomic DNA sequence of SEQ ID NO: 6, e indicates a phage having the genomic DNA sequence of SEQ ID NO: 7, f indicates the 1st phage having the genomic DNA sequence of SEQ ID NO: 1, g indicates the 3rd phage having the genomic DNA sequence of SEQ ID NO: 3, and h indicates a phage having the genomic DNA sequence of SEQ ID NO: 8. In the diagram, the mark "/" indicates that the phages before and after the mark are used in combination. For example, "a/f" indicates that the 2nd phage and the 1st phage are used in combination.

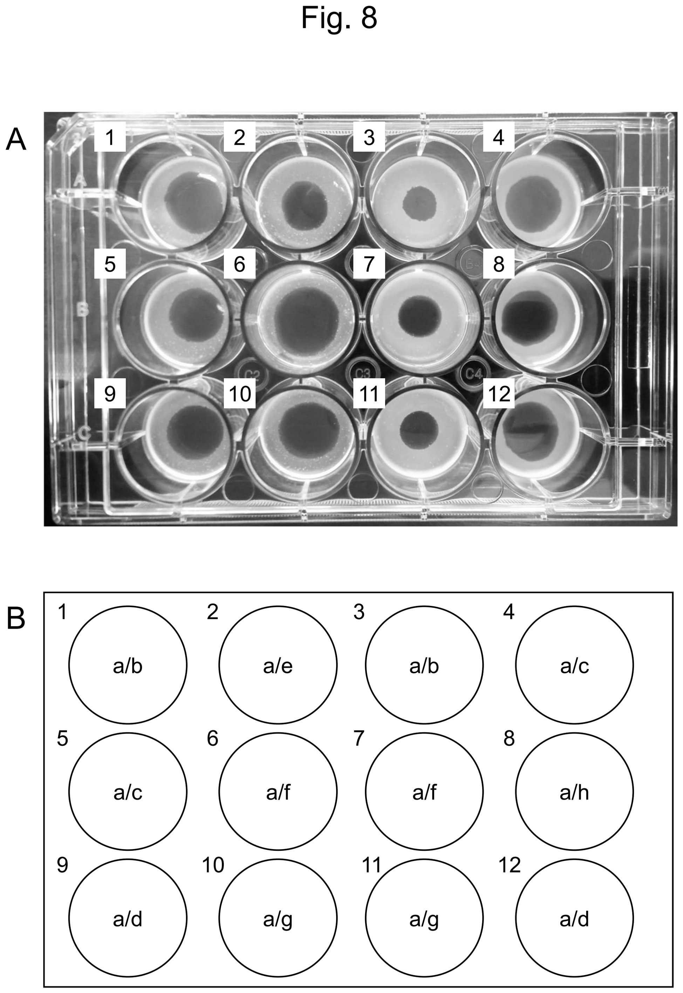

[Figure 8] Figure 8 shows the bacteriolytic ability of the combinations of the 2nd bacteriophage and another bacteriophage tested in Example 6. Figure 8A shows a static culture on an agar plate on which the bacteria of the *Xanthomonas* genus were spread and cultured, and onto which a refined solution of phage was then dropped. Figure 8B is a diagram of the plates corresponding to Figure 8A, showing the kinds of phages comprised in the refined solutions of phages dropped onto the respective plates. In Figures 8A and 8B, the left two columns (1, 2, 5, 6, 9 and 10) each indicate a plate on which the bacteria with the ID MAFF No. 673005 was spread, the 3rd column from the left (3, 7 and 11) indicates a plate on which the bacteria with the ID MAFF No. 301257 was spread, the rightmost column (4, 8 and 12) indicates a plate on which the bacteria with the ID MAFF No. 106765 was spread. In Figure 8B, a indicates the 3rd phage having the genomic DNA sequence of SEQ ID NO: 3, b indicates a phage having the genomic DNA sequence of SEQ ID NO: 4, c indicates a phage having the genomic DNA sequence of SEQ ID NO: 5, d indicates a phage having the genomic DNA sequence of SEQ ID NO: 6, e indicates a phage having the genomic DNA sequence of SEQ ID NO: 7, f indicates the 1st phage having the genomic DNA sequence of SEQ ID NO: 1, g indicates the 2nd phage having the genomic DNA sequence of SEQ ID NO: 2, and h indicates a phage having the genomic DNA sequence of SEQ ID NO: 8. In the diagram, the mark "/" indicates that the phages before and after the mark are used in combination. For example, "a/f" indicates that the 3rd phage and the 1st phage are used in combination.

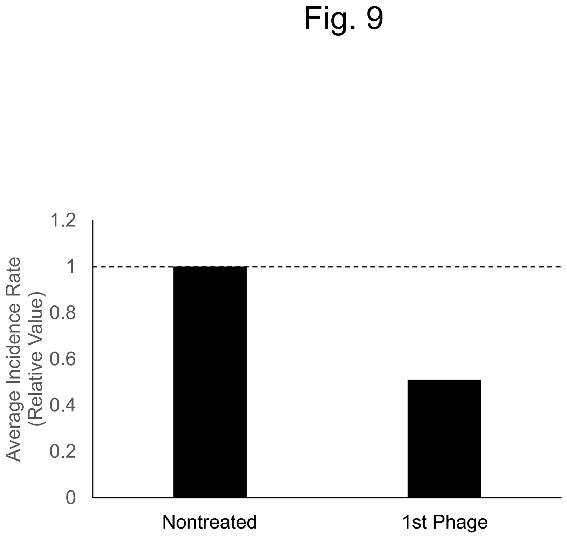

[Figure 9] Figure 9 graphs the result of a disease control effect test on a tomato bacterial blight tested in Example 7. The average incidence rate is expressed as a relative value with respect to a nontreated group.

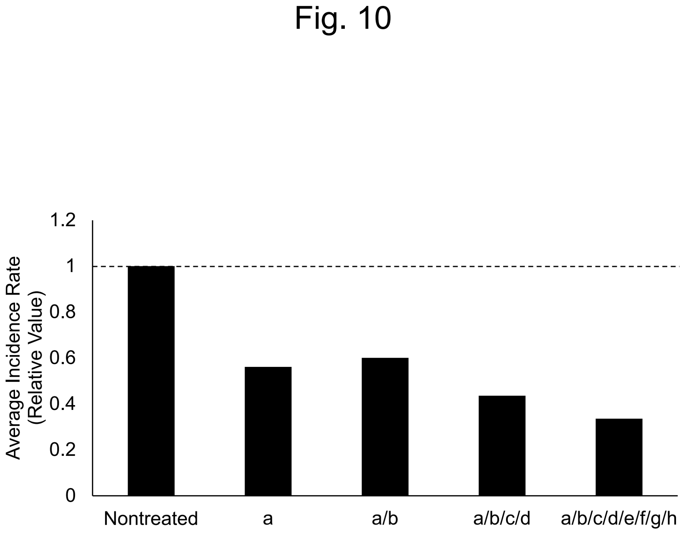

[Figure 10] Figure 10 graphs the result of a disease control effect test on a black rot disease of broccoli tested in Example 8. The average incidence rate is expressed as a relative value with respect to a nontreated group. The number below each bar indicates the kind of phage used. In the graph, a indicates the 3rd phage having the genomic DNA sequence of SEQ ID NO: 3, b indicates a phage having the genomic DNA sequence of SEQ ID NO: 9, c indicates a phage having the genomic DNA sequence of SEQ ID NO: 8, d indicates a phage having the genomic DNA sequence of SEQ ID NO: 10, e indicates a phage having the genomic DNA sequence of SEQ ID NO: 5, f indicates a phage having the genomic DNA sequence of SEQ ID NO: 6, g indicates a phage having the genomic DNA sequence of SEQ ID NO: 7, and h indicates a phage having the genomic DNA sequence of SEQ ID NO: 11. In the diagram, the mark "/" indicates that the phages before and after the mark are used in combination. For example, "a/b" indicates that the 3rd phage and the phage having the genomic DNA sequence of SEQ ID NO: 9 are used in combination.

### Description of Embodiments

### 1. Bacteriolytic Agent

### 1-1. Overview

The first aspect of the present invention is a bacteriolytic agent. A bacteriolytic agent of the present invention consists of a bacteriophage having a genomic sequence comprising a specific nucleotide sequence.

A bacteriolytic agent of the present invention exhibits bacteriolytic ability specifically against target bacteria that can be pathogenic bacteria for a plant disease.

### 1-2. Definition

Terms used herein are defined below.

A "bacteriolytic agent" as used herein refers to a drug consisting of a bacteriophage having bacteriolytic ability against target bacteria.

"Bacteria", besides archaea and eukaryotes, is one of the three major biological lineages into which the whole biological world is classified. A bacterium is composed of a cell without a nucleus (acaryote) and can self-reproduce itself with a nutrient source. Bacteria are named with a genus and a species under a family in accordance with the International Code of Nomenclature of Bacteria.

"Target bacteria" as used herein refers to host bacteria that may be a target for a phage constituting a bacteriolytic agent of the present invention or a phage comprised in a composition for plant disease control and a composition according to the present invention. An example is a bacterium having a membrane surface receptor to be recognized on the outer membrane (epicyte) by the phage. A "membrane surface receptor" is a site to which, for example, a tail, a tail fiber, and the like of a phage are bound, and is composed of a protein, a lipopolysaccharide, pili, or the like that is present in the outer layer of the bacterial outer membrane. A specific example of the target bacteria herein is bacteria of the *Xanthomonas* genus.

"Bacteria of the *Xanthomonas* genus" refers to bacteria belonging to the *Xanthomonas* genus. Bacteria of the *Xanthomonas* genus commonly produce a yellow pigment called Xanthomonasin, and many of the bacteria are known to be plant pathogenic bacteria. In this connection, there are subtypes and pathovars as subclasses of species, and they are indicated *by subsp.* or pv. following the name of the bacteria, respectively. Additionally, the smallest unit of classification is a strain, which refers to a cell population considered to be genetically uniform. Table 1 below shows typical bacteria of the *Xanthomonas* genus, host plants thereof, and plant diseases caused thereby.

**[Table 1]**

| **Species** | **Pathovar** | **Host** | **Disease** |
|---|---|---|---|
| ***X. arboricola*** | *pruni* | Peach | Bacterial spot |
| ***X. arboricola*** | *juglandis* | Walnut | Bacterial blight |
| ***X. arboricola*** | *corylina* | Turkish Hazel | Leaf spot |
| ***X. axonopodis*** | *citrumelo* | Citrus | Bacterial spot |
| ***X. axonopodis*** | *glycines* | Soybean | Bacterial pustule |
| ***X. axonopodis*** | *manihotis* | Cassava | Bacterial blight |
| *X. **axonopodis*** | *malvacearum* | Cotton | Bacterial blight |
| ***X. axonopodis*** | *punicae* | Pomegranate | Leaf blight |
| ***X. albilineans*** | | Sugarcane | Leaf scald |
| ***X. campestris*** | *campestris* | Cabbage | Black rot |
| ***X. campestris*** | *musacearum* | Banana | Bacterial wilt |
| ***X. campestris*** | *vasculorum* | Sugarcane | Gumming disease |
| ***X. campestris*** | *vesicatoria* | Tomate/Pepper | Bacterial spot |
| ***X. campestris*** | *vitians* | Lettuce | Bacterial spot |
| ***X. campestris*** | *raphani* | Cabbage | Leaf spot |
| ***X. citri*** | *citri* | Orange | Bacterial canker |
| ***X. citri*** | *malvacearum* | Cotton | Angular leaf spot |
| ***X. citri*** | *mangiferaeindicae* | Mango | Black spot |
| ***X. cucurbitae*** | | Pumpkin | Bacterial Spot |
| ***X. fragariae*** | | Strawberry | Angular leaf spot |
| **X. *fuscans*** | *fuscans* | Bean | Bacterial blight |
| ***X. hortorum*** | *carotae* | Carrot | Bacterial blight |
| **X. *oryzae*** | *oryzae* | Rice | Leaf blight |

Among the bacteria of the *Xanthomonas* genus, *Xanthomonas arboricola, Xanthomonas citri,* and *Xanthomonas campestris* are particularly preferable as target bacteria in the present invention, without limitation. Specific examples of *Xanthomonas arboricola* include: *Xanthomonas arboricola pv. pruni,* the pathovar of which is *pruni;* and *Xanthomonas arboricola pv. juglandis,* the pathovar of which is *juglandis.* Specific examples of *Xanthomonas citri* include *Xanthomonas citri subsp. citri.* Specific examples of *Xanthomonas campestris* include: *Xanthomonas campestris pv. vesicatoria,* the pathovar of which is *vesicatoria; Xanthomonas campestris pv. vitians,* the pathovar of which is *vitians;* and *Xanthomonas campestris pv. campestris,* the pathovar of which is *campestris.*

A "bacteriophage" (herein often abbreviated simply as a "phage," as mentioned above) is a generic term for viruses that infect bacteria. A common phage is composed of three parts: a head (head part), a tail (tail part), and a tail fiber (tail fiber part). The head, constituted by a capsomere that is a coat protein, is composed of a capsid (viral shell) having an icosahedral structure, in the inner space of which the genomic DNA of a phage is encapsulated. The tail has a tubular structure composed of a tail tubular protein and a sheath protein covering the same. One end and the other of the tail are linked to the head and the tail fiber, respectively. The tail functions as an introduction tube through which the genomic DNA in the head is injected into the cell of the host bacteria. The tail and the tail fiber have a structure of several fibers composed of a tail fiber protein. The tail and the tail fiber serve a host-recognizing function and an attachment function, i.e., to recognize a receptor on the surface of the outer membrane of host bacteria and be attached to the cell surface. A phage has an extremely high host specificity, and this characteristic is based on the functions of the tail and the tail fiber. A phage does not infect a eukaryote, and thus, a drug comprising a phage is harmless to humans, animals, and plants. In this connection, phages are roughly classified into a "bacteriolytic cycle", a "lysogenic cycle", and a "bacteriolytic/lysogenic cycle" according to the mode of infection. In the lysogenic cycle, a phage incorporates (introduces) its own DNA into a chromosome of target bacteria without bacteriolyzing the bacteria and proliferates as the bacteria proliferate. On the other hand, in the bacteriolytic cycle, a phage self-proliferates in a cell of host bacteria and then bacteriolyzes the host bacteria to release a large number of daughter phages. As a phage in the present invention, a virulent phage that undergoes the bacteriolytic cycle is intended.

A "tail fiber protein" is a protein constituting the tail fiber of a phage, as described above. It is known that the tail fiber protein plays an important role in the specificity of the host recognition and attachment capability of the tail and the tail fiber (Nobrega F.L. et al., Nat. Rev. Microbiol., 2018, 16: 760-773).

A "tail fiber gene" refers to a gene encoding the tail fiber protein comprised in the genomic DNA of a phage.

A "tail tubular protein" is a protein constituting the tubular structure of the tail of a phage, as described above. It is known that the tail tubular protein interacts with the tail fiber and, together with the tail fiber, plays an important role in the specificity of the host recognition and attachment capability (Maozhi Hu, *et al.,* 2020, 9:1, 855-867). As tail tubular proteins, a tail tubular fiber protein A and a tail tubular protein B are known. The "tail tubular protein A" is a protein that forms a ring at the lower part of the tubular structure of the tail and interacts with the tail fiber. The "tail tubular protein B" is a protein that forms the end of the lower part of the tubular structure of the tail and is bound to a receptor on the surface of the outer membrane of host bacteria.

A "tail tubular gene" refers to a gene encoding the tail tubular protein comprised in the genomic DNA of a phage. The "tail tubular protein A gene" and the "tail tubular protein B gene" refer to a gene encoding the tail tubular protein A and a gene encoding the tail tubular protein B, respectively.

"Bacteriolysis" refers to a phenomenon that destroys the cell membrane of bacteria. As described above, the phenomenon is mainly observed in the mode of infection of a virulent phage. Bacteriolysis kills bacteria. Bacteriolysis starts when a phage is specifically attached to a target bacterium and injects its own DNA into a cell of the target bacterium via the tail. Then, the phage utilizes the translational mechanism of the bacteria for self-reproduction to produce a large number of daughter phages and then bacteriolyzes the bacteria to release the daughter phages to the outer space.

A "plant disease" as used herein is a generic term for diseases caused in a plant. A known plant disease includes a disease caused by an infectious pathogen such as a virus, bacteria, filamentous fungus, actinomycete, viroid, *Phytoplasma,* nematode, mite, or insect, or a disease caused by a noninfectious pathogen such as the lack or excess of a nutrient or water, drug poisoning, or the like. A plant disease herein refers to a disease whose pathogens are bacteria, i.e., a plant pathogenic bacterium, unless otherwise specified. A plant pathogenic bacterium herein corresponds to the above-described target bacteria, for example, bacteria of the *Xanthomonas* genus, unless otherwise specified.

"Control" as used herein refers to prevention or treatment (extermination) (from the home page of the Japan Crop Protection Association). Accordingly, "plant disease control" as used herein refers to the prevention of a plant disease, particularly against a target bacterium, or to the treatment of a plant disease caused by a target bacterium.

A "target plant" as used herein refers to a plant to which the below-described composition for plant disease control according to the present invention is to be applied. This plant is a plant that has a specific plant disease caused by the infection of the target bacteria, or a plant that has the possibility of being infected by the target bacteria.

### 1-3. Constitution

A bacteriolytic agent of the present invention consists of a bacteriophage.

### <1st Phage>

The 1st phage is characterized by having a genomic DNA sequence comprising a specific nucleotide sequence, and a bacteriolytic agent of the present invention exhibits specific bacteriolytic ability against target bacteria.

### (1-1) Genomic DNA

The genomic DNA sequence of the 1st phage has: the 42,933 bp nucleotide sequence of SEQ ID NO: 1; the nucleotide sequence having addition, deletion, and/or substitution of one or a plurality of nucleotides in the nucleotide sequence of SEQ ID NO: 1; a nucleotide sequence having a sequence identity of 92.15% or more, 92.2% or more, 92.3% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, or 99.5% or more to the nucleotide sequence of SEQ ID NO: 1; a nucleotide sequence having a sequence identity of 99.01% or more, 99.05% or more, 99.1% or more, 99.2% or more, 99.3% or more, 99.4% or more, 99.5% or more, 99.6% or more, 99.7% or more, 99.8% or more, or 99.9% or more when aligned with respect to the nucleotide sequence of SEQ ID NO: 1; or furthermore a nucleotide sequence that hybridizes with the nucleotide sequence complementary to the nucleotide sequence of SEQ ID NO: 1 under highly stringent conditions.

For a very long genomic DNA sequence, it is not easy to set the whole range as the range to be compared by alignment. The range to be compared (hereinafter, also referred to as Query Cover) is most preferably the range of 100% of the whole genome of the 1st phage and may be 99% or more, 98% or more, 97% or more, 96% or more, 95% or more, 94% or more, 93.5% or more, or 93% or more thereof.

"A plurality" as used herein refers to 2 to 10, for example, 2 to 7, 2 to 5, 2 to 4, or 2 to 3.

A "(nucleotide) sequence identity" as used herein is a value that indicates the ratio of the sites at which the kinds of nucleotides are the same in a range to be compared between two nucleotide sequences. Even when the two nucleotide sequences are different in length, the nucleotide sequence identity can be calculated by aligning the nucleotides so that the degree of coincidence of the nucleotides in a range to be compared becomes the highest. Without limitation, a typical algorithm for such an analysis is BLAST. BLAST may be utilized with various software or web services. A nucleotide sequence identity may be calculated easily, utilizing, for example, genetic information processing software GENETYX (https://www.genetyx.co.jp/) or the NCBI-provided BLAST server (https://blast.ncbi.nlm.nih.gov/Blast.cgi). Besides BLAST, an algorithm called FASTA, or the like, may be utilized, and the method used is not particularly limited, as long as it is capable of calculating an appropriate identity.

The "highly stringent conditions" refer to environmental conditions that are hardly prone to nonspecific hybridization. Under highly stringent conditions, a hybrid can be formed with a nucleic acid having a target nucleotide sequence, but a hybrid cannot substantially be formed with a nucleic acid having a nonspecific nucleotide sequence. Generally, highly stringent conditions refer to low-salt-concentration and high-temperature conditions. The low-salt concentration is, for example, 15 mM to 750 mM, preferably 15 mM to 500 mM, 15 mM to 300 mM, or 15 mM to 200 mM. Additionally, the high temperature is, for example, 50 to 68°C or 55 to 70°C. Specific examples of highly stringent conditions include conditions where hybridization is followed by washing with 0.1× SSC and 0.1% SDS at 65°C.

From another viewpoint, the phage is a virus, and the possibility may be taken into consideration that a mutation such as substitution, deletion, or insertion occurs in the genomic DNA when the phage of the present invention is amplified. Even such a mutation, if any, is included in the scope of the present invention as long as the degree of the mutation falls within the aforementioned range with respect to the whole genomic DNA and the bacteriolysis function of the phage is maintained.

The software and analysis server may indicate a sequence identity according to the index, such as Average Nucleotide Identity (ANI), and these may also be used. In this connection, for a very long phage genomic DNA, it is not easy to set the whole range as the range to be compared by alignment. Accordingly, the above-described sequence identity may be applicable for the range to be aligned automatically provided by the above-mentioned software or Web service. For example, in an analysis using the NCBI-provided BLAST server, a Query sequence and a Subject sequence are aligned automatically in the maximum alignable range so that a range to be compared is determined. The sequence identity in this range to be compared is calculated, while the ratio of the range to be compared out of the whole range of the Query sequence may be calculated as a value called Query Cover.

The value may vary depending on how to select the Query sequence and the Subject sequence. This point will be described briefly by taking, as an example, the 1st sequence composed of a sequence A and the 2nd sequence composed of the sequence A and a sequence B having the same length thereas. When the shorter 1st sequence is used as the Query sequence and the longer 2nd sequence is used as the Subject sequence, the range to be compared is the sequence A, and the value of Query Cover to be calculated is 100% because the sequence A is the full length of the 1st sequence. Since the 1st sequence and the 2nd sequence have the same sequence A, the value of sequence identity is 100%. On the other hand, when the longer 2nd sequence is rather used as the Query sequence and the shorter 1st sequence is used as the Subject sequence, the value of sequence identity is 100%, whereas the value of Query Cover is 50% because the sequence A occupies only half the 2nd sequence.

In such a case, the result may be used as a basis to estimate the sequence identity of the nucleotide sequence in the whole range of the aligned nucleotide sequence (the sequence identity of the whole range). For example, a value obtained by multiplying a Query Cover value by the value of a sequence identity in the range to be compared after alignment (the sequence identity in the range to be aligned) may be used as the estimated value of the sequence identity of the whole range. In this case, to increase the accuracy of the estimated value, a further correction may be added. For example, a sequence identity expected for a range other than the range to be aligned may be reckoned in.

In this regard, the genomic DNA of a phage is packaged linearly or circularly. Additionally, in a next-generation genome sequencer analysis, the genomic DNA is fragmented, the nucleotide sequence of each of the fragments is read, and an analysis for connecting the sequences is performed to determine a sequence. In the case of a phage, the sequences are often connected without a reference genomic DNA sequence (de novo assembly). Therefore, it is difficult to unambiguously determine the start and end of a genome to be analyzed (Merrill, B.D., *et al. BMC Genomics,* 2016 17, 679). The starts and ends of genomic sequences in comparison may differ and are automatically taken into consideration in an analysis using software or an analysis server.

Among the bacteria of the *Xanthomonas* genus, *Xanthomonas arboricola, Xanthomonas citri,* and *Xanthomonas campestris* are preferable as target bacteria for the 1st phage, without particular limitation. Specific examples of *Xanthomonas arboricola* suitable as target bacteria for the 1st phage include: *Xanthomonas arboricola pv. pruni,* the pathovar of which is *pruni;* and *Xanthomonas arboricola pv. juglandis,* the pathovar of which is *juglandis.* Specific examples of *Xanthomonas citri* suitable as target bacteria for the 1st phage include *Xanthomonas citri subsp. citri.* Specific examples of *Xanthomonas campestris* suitable as target bacteria for the 1st phage include: *Xanthomonas campestris pv. vesicatoria,* the pathovar of which is *vesicatoria;* and *Xanthomonas campestris pv. vitians,* the pathovar of which is *vitians.*

### (1-2) Effects

A bacteriolytic agent of the present invention comprising the 1st phage can exhibit bacteriolytic ability against bacteria of the *Xanthomonas* genus and can be used for plant diseases.

### <2nd Phage>

The 2nd phage is characterized by having a genomic DNA sequence with a specific length comprising a specific nucleotide sequence, and a bacteriolytic agent of the present invention exhibits specific bacteriolytic ability against target bacteria.

### (2-1) Genomic DNA

The genomic DNA sequence of the 2nd phage has: the 35,321 bp nucleotide sequence of SEQ ID NO: 2; the nucleotide sequence having addition, deletion, and/or substitution of one or a plurality of nucleotides in the nucleotide sequence of SEQ ID NO: 2; a nucleotide sequence having a sequence identity of 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, or 99.5% or more to the nucleotide sequence of SEQ ID NO: 2; a nucleotide sequence having a sequence identity of 95.0% or more, 95.5% or more, 96.0% or more, 96.5% or more, 97.0% or more, 97.5% or more, 98.0% or more, 98.5% or more, 99% or more, 99.5% or more, 99.6% or more, 99.7% or more, 99.8% or more, or 99.9% or more when aligned with respect to the nucleotide sequence of SEQ ID NO: 2; or furthermore a nucleotide sequence that hybridizes with the nucleotide sequence complementary to the nucleotide sequence of SEQ ID NO: 2 under highly stringent conditions.

For a very long genomic DNA sequence, it is not easy to set the whole range as the range to be compared. The range to be compared (hereinafter, also referred to as Query Cover) is most preferably the range of 100% of the genomic full length of the 2nd phage and may be 99% or more, 98% or more, 97% or more, 96% or more, 95% or more, 94% or more, or 93% or more thereof.

The genomic DNA sequence of the 2nd phage has a full length of 40,000 or less nucleotides. For example, the full length of the genomic DNA sequence is 40,000 or less nucleotides, 39,500 or less nucleotides, 39,000 or less nucleotides, 38,500 or less nucleotides, 38,000 or less nucleotides, 37,500 or less nucleotides, 37,000 or less nucleotides, 36,500 or less nucleotides, 36,000 or less nucleotides, 35,900 or less nucleotides, 35,800 or less nucleotides, 35,700 or less nucleotides, 35,600 or less nucleotides, 35,500 or less nucleotides, or 35,400 or less nucleotides.

Among the bacteria of the *Xanthomonas* genus mentioned above in the section "Definition", *Xanthomonas arboricola* and *Xanthomonas campestris* are particularly preferable as target bacteria for the 2nd phage, without particular limitation. Specific examples of *Xanthomonas arboricola* suitable as target bacteria for the 2nd phage include: *Xanthomonas arboricola pv. pruni,* the pathovar of which is *pruni;* and *Xanthomonas arboricola pv. juglandis,* the pathovar of which is *juglandis.* Specific examples of *Xanthomonas campestris* suitable as target bacteria for the 2nd phage include: *Xanthomonas campestris pv. vesicatoria,* the pathovar of which is *vesicatoria; Xanthomonas campestris pv. vitians,* the pathovar of which is *vitians;* and *Xanthomonas campestris pv. campestris,* the pathovar of which is *campestris.*

### (2-2) Effects

A bacteriolytic agent of the present invention comprising the 2nd phage can exhibit bacteriolytic ability against bacteria of the *Xanthomonas* genus and can be used for plant diseases.

### <3rd Phage>

The 3rd phage is characterized by having a genomic DNA sequence comprising a specific nucleotide sequence, and a bacteriolytic agent of the present invention exhibits specific bacteriolytic ability against target bacteria.

### (3-1) Genomic DNA

The genomic DNA sequence of the 3rd phage has: the 43,601 bp nucleotide sequence of SEQ ID NO: 3; the nucleotide sequence having addition, deletion, and/or substitution of one or a plurality of nucleotides in the nucleotide sequence of SEQ ID NO: 3; a nucleotide sequence having a sequence identity of 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, or 99.5% or more to the nucleotide sequence of SEQ ID NO: 3; a nucleotide sequence having a sequence identity of 96.9% or more, 97.0% or more, 97.5% or more, 98.0% or more, 98.5% or more, 99% or more, 99.5% or more, 99.6% or more, 99.7% or more, 99.8% or more, or 99.9% or more when aligned with respect to the nucleotide sequence of SEQ ID NO: 3; or furthermore a nucleotide sequence that hybridizes with the nucleotide sequence complementary to the nucleotide sequence of SEQ ID NO: 3 under highly stringent conditions.

For a very long genomic DNA sequence, it is not easy to set the whole range as the range to be compared. The range to be compared (hereinafter, also referred to as Query Cover) is most preferably the range of 100% of the genomic full length of the 3rd phage and may be 99% or more, 98% or more, 97% or more, 96% or more, 95% or more, 94% or more, 93% or more, 92% or more, 91% or more, 90% or more, 89% or more or 88% or more thereof.

Among the bacteria of the *Xanthomonas* genus mentioned above in the section "Definition", *Xanthomonas arboricola* and *Xanthomonas campestris* are preferable as target bacteria for the 3rd phage, without particular limitation. Specific examples of *Xanthomonas arboricola* suitable as target bacteria for the 3rd phage include *Xanthomonas arboricola pv. juglandis,* the pathovar of which is *juglandis.* Specific examples of *Xanthomonas campestris* suitable as target bacteria for the 3rd phage include: *Xanthomonas campestris pv. vesicatoria,* the pathovar of which is *vesicatoria; Xanthomonas campestris pv. vitians,* the pathovar of which is *vitians;* and *Xanthomonas campestris pv. campestris,* the pathovar of which is *campestris.*

### (3-2) Effects

A bacteriolytic agent of the present invention comprising the 3rd phage can exhibit bacteriolytic ability against bacteria of the *Xanthomonas* genus and can be used for a plant disease.

### 2. Composition for Plant Disease Control

### 2-1. Overview

A second aspect of the present invention is a composition, particularly a composition usable for plant disease control. A composition of the present invention is characterized by comprising the bacteriolytic agent according to the first aspect as an active component.

Using the composition of the present invention for plant disease control can provide sustainable plant protectives (agricultural chemicals) against a bacterial plant disease, wherein the protectives are safe for the human body, free from drug poisoning to the environment, and capable of preventing or treating a specific plant disease of interest.

In this regard, the expression "composition for plant disease control" as used herein refers to the composition of the present invention used for plant disease control.

### 2-2. Constitution

### 2-2-1. Component

A composition of the present invention comprises, as an essential component, a bacteriophage that is a bacteriolytic agent described in the first aspect as an active component. Additionally, the composition may comprise an agriculturally acceptable carrier and/or medium to the extent that the bacteriolytic ability of the phage to target bacteria is not impaired or inhibited. Furthermore, the composition may comprise other active components, if desired. Each component is specifically described below.

### (1) Active Component (Bacteriolytic Agent)

A composition of the present invention comprises the bacteriolytic agent according to the first aspect as an essential active component. Because this active component bacteriolyzes the target bacteria of the present invention, the composition for plant disease control can prevent or treat a plant disease caused by the target bacteria.

The specific constitution of the bacteriolytic agent has been described in detail in the first aspect and is thus omitted here.

When the composition is used for plant disease control, the amount of the active component comprised per unit amount in the composition depends on various conditions, such as the dosage form, the kind of plant pathogenic bacteria, the kind of target plant, the site of application, and the method of application. The amount to be comprised is preferably sufficient for the phage, as an active component, to contact and infect plant pathogenic bacteria that have infected a target plant. Accordingly, it can be determined within the scope of common technical knowledge in the art, considering the conditions such that the bacteriolytic agent comprised in a composition for plant disease control according to the present invention is in an effective amount for target bacteria after application.

### (2) Agriculturally Acceptable Carrier and Medium

An "agriculturally acceptable carrier and/or medium" refers to a substance that facilitates the application of a composition, can maintain the survivability and infectiousness of a phage as an active component, and/or can control the rate of action, and that, when applied outdoor, has no or an extremely small harmful influence on an environment such as the soil and the water quality, and further has no or an extremely low harmfulness to an animal, particularly a human.

### (2-1) Carrier

Specific examples of agriculturally acceptable carriers include surfactants, protective agents, and excipients. Additionally, if desired, a wetting agent, emulsifying agent, pH buffering agent, and the like may be utilized in small amounts. The carrier may be blended in advance or may be blended immediately before application.

The surfactant has an effect on enhancing the physicochemical properties of the composition with respect to parts of a plant, the properties including hygroexpansivity (wetting capability), emulsifying capability, dispersibility, permeating capability, adhesiveness, defoaming capability, and spreading capability. The surfactant may be utilized as a main component of an agricultural adjuvant called a spreading agent. Examples of spreading agents include nonionic surfactants, combinations of a nonionic surfactant and an anionic surfactant, paraffins, and polyoxyethylene resin acid esters. More specific examples include polyoxyethylenealkyl ether compounds, polyoxyethylene fatty acid ester compounds, lignin sulfonic acid salt compounds, naphthylmethanesulfonic acid salt compounds, alkylsulfosuccinic acid salt compounds, and tetraalkylammonium salt compounds.

A protective agent for a phage is expected to have an effect, such as decreasing damage due to ultraviolet rays. Examples include skim milks, caseins, and gelatins.

Examples of excipients include glucose, lactose, sucrose, gelatin, starch, malt, and flour.

### (2-2) Solvent

Specific examples of agriculturally acceptable solvents include water (including an aqueous solution), buffers, and liquid culture media. The solvent is, preferably, an aseptic liquid.

### (3) Another Active Component

A composition of the present invention may comprise, in addition to a bacteriolytic agent according to the first aspect, one or more other active components having the same and/or different pharmacologic actions to the extent that the component(s) does/do not affect the bacteriolytic ability of the phage constituting the bacteriolytic agent.

The (an)other active component(s) is/are not limited to any kind. For example, a phage, which may have bacteriolytic ability against the same and/or different bacteria, may be used. Examples of the phage having bacteriolytic ability against the same bacteria include another phage that specifically recognizes and is attached to bacteria of the *Xanthomonas* genus in the same manner as a phage constituting a bacteriolytic agent according to the first aspect. For example, even if target bacteria are the same between phages, the phages, if capable of recognizing different cell surface receptors, can be expected to allow a synergistic effect or supportive effect of the bacteriolytic ability, depending on the combination.

Such other phages that specifically recognize bacteria of the *Xanthomonas* genus and have bacteriolytic ability are not particularly limited by their types. Specific examples thereof include a phage, the genomic DNA of which comprises the nucleotide sequence of any one of SEQ ID NOs: 4 to 11, and a variant and an engineered form thereof.

A composition for plant disease control of the present invention may comprise, in addition to a bacteriolytic agent according to the first aspect, as an active component(s) in combination, one or more other phages that specifically recognize bacteria of the *Xanthomonas* genus and have bacteriolytic ability, as specifically exemplified above.

In the above description, the sequence identity of the amino acid sequence is suitably 95% or more, 96% or more, 97% or more, 98% or more, or 99% or more. As already described, "a plurality" as used herein refers to 2 to 10, for example, 2 to 7, 2 to 5, 2 to 4, or 2 to 3.

Additionally, a "substitution (of an amino acid)" refers to a substitution within a group of conservative amino acids, in which the amino acids are similar in properties such as the electric charge, side chain, polarity, and aromaticity among the 20 kinds of amino acids constituting natural proteins. The substitution is, for example, within the uncharged polarity amino acid group having a low-polarity side chain (Gly, Asn, Gln, Ser, Thr, Cys, and Tyr), the branched-chain amino acid group (Leu, Val, and Ile), the neutral amino acid group (Gly, Ile, Val, Leu, Ala, Met, and Pro), the neutral amino acid group having a hydrophilic side chain (Asn, Gln, Thr, Ser, Tyr, and Cys), the acidic amino acid group (Asp and Glu), the basic amino acid group (Arg, Lys, and His), and the aromatic amino acid group (Phe, Tyr, and Trp). An amino acid substitution within such a group is known to be less likely to change the properties of a polypeptide, and thus, is preferable.

Furthermore, an "amino acid sequence identity" as used herein is a value that indicates the ratio of the sites at which the kinds of amino acid residues are the same in a range to be compared between two amino acid sequences. Even when the two amino acid sequences are different in length, the amino acid sequence identity can be calculated by aligning the amino acids so that the degree of coincidence of the amino acids in a range to be compared becomes the highest. Without limitation, the already described algorithm, such as BLAST, may be utilized as an algorithm for such an analysis.

As other examples, insecticides, herbicides, fertilizers (for example, urea, ammonium nitrate, and perphosphate), and, if desired, known chemical pesticides, antibiotics, and biological agricultural chemicals may be comprised as another active component.

### 2-2-2. Dosage Form

The dosage form of a composition of the present invention, when the composition is used for plant disease control, may be any dosage form, as long as it is possible that a site of infection of target bacteria on a target plant, the fixability to a target plant, and/or the easiness of infection of a phage as an active component against target bacteria is maintained. For example, the composition for plant disease control may be suspended in a suitable solution to be formed into a liquid agent or a dispersible (water-dispersible) agent, or may be mixed with a carrier and solidified to be formed into a solid-state powder, granule, or gel. For example, when the site of infection of target bacteria on a target plant is a leaf, flower, fruit, stem, branch, or trunk in the aerial part, a liquid, dispersible agent, or gel, which widely spreads in such a site of infection, and has high fixability, is suitable, without limitation. On the other hand, when the site of infection of target bacteria is a root or an underground stem in the underground part, a powder or granule, that is released in a sustained manner in soil, and can sustainably achieve an effect at the site of infection, is suitable, without limitation.

### 2-3. Method for Application

When a composition of the present invention is used as a composition for plant disease control, a method for applying the composition may be a method known in the art, and is not particularly limited, as long as it is possible to apply the composition for plant disease control to a target plant. A phage as an active component of a composition for plant disease control can intrude through any part of the whole surface of the target plant, such as the stem-and-leaf part and the root part, and thus, may be applied by a suitable method in accordance with the purpose. For example, when the site of application is in an aerial part, such as a stem-and-leaf part, the composition for plant disease control may be applied so as to come into direct contact with the site of application. The direct contact involves, for example, painting, spraying, scattering, or immersing the composition for plant disease control at the site of application. The application is particularly preferably performed to the site, where it is infected or potentially infected by target bacteria, of a target plant. Additionally, the application may be indirect through soil when the site of application is in an underground part, such as a root, or by addition to a culture medium when the site is in the medium. "Soil" as used herein is not particularly limited, as long as it is possible to grow a target plant. Usually, a planting soil comprising a suitable nutrient and having a suitable pH value is utilized. The site of the soil is not limited. Additionally, a "culture medium" refers to a planting medium artificially prepared for a target plant. The medium may be a solid medium, such as an agar medium, or may be a liquid medium. The culture medium is, for example, an isolating bed, a root zone restriction pot, or a nursery. The composition of the culture medium may be a medium composition known in the art. The composition may be suitably selected according to the kind of plant, or the like.

### 2-4. Target Plant

A target plant for a composition for plant disease control according to the present invention is not particularly limited to any kind, as long as it is possible to develop a plant disease caused by the target bacteria of the present invention. The target plant may be either an angiosperm or a gymnosperm. Furthermore, the plant may be an herbaceous plant or a woody plant. Suitable specific examples of the target plant include agriculturally important plants, for example: crop plants such as cereal crops, vegetables, and fruits; and flowers and ornamental plants. Specific examples include monocotyledons, such as Poaceae plants (for example, rice, wheat, barley, corn, sugarcane, *Sorghum,* kaoliangs, and turfgrasses), Musaceae plants (for example, bananas), Amaryllidaceae plants (for example, *Allium,* onions, garlic, and garlic chives), and Liliaceae plants (for example, lilies and tulips). Additional examples include dicotyledons, such as Brassicaceae plants (for example, cabbages, radishes, Chinese cabbages, and rapeseeds), Asteraceae plants (for example, lettuces, burdocks, and *Chrysanthemum),* Fabaceae plants (for example, soybeans, peanuts, garden peas, phaseoli, lentils, chickpeas, fava beans, and licorice), Solanaceae plants (for example, tomatoes, eggplants, potatoes, tobaccos, bell peppers, red peppers, and petunia), Rosaceae plants (for example, strawberries, apples, pears, peaches, *Eriobotrya,* almonds, plums, roses, Japanese apricots, and cherries), Cucurbitaceae plants (for example, cucumbers, gourd, pumpkins, melons, and watermelons), Anacardiaceae plants (for example, mangoes, pistachios, and cashew nuts), Lauraceae plants (for example, avocadoes) Rutaceae plants (for example, mandarin oranges, grapefruits, lemons, and *Citrus junos),* Convolvulaceae plants (for example, sweet potatoes), Theaceae plants (for example, *Camellia sinensis),* and Vitaceae plants (for example, grapes).

### 2-5. Target Plant Disease

A target plant disease to which a composition for plant disease control according to the present invention is to be applied is any plant disease that is caused by the target bacteria of the present invention. The disease is, preferably, a plant disease caused by bacteria of the *Xanthomonas* genus. Examples include: bacterial spot observed in peaches and the like; bacterial blight observed in walnuts and the like; bacterial pustule observed in soybeans and the like; angular leaf spot observed in strawberries and the like; bacterial blight observed in tomatoes, bell peppers, lettuces, and the like; black rot observed in cabbages, Chinese cabbages, broccoli, and the like; bacterial canker observed in oranges, grapefruit, and the like; angular leaf spot observed in cotton and the like; and leaf blight observed in rice and the like.

### 3. Method for Controlling Plant Disease

### 3-1. Overview

A third aspect of the present invention is a method for controlling plant disease. A method for controlling plant disease according to the present invention is characterized by applying a composition for plant disease control according to the second aspect to a target plant to control a plant disease of the target plant.

A method for controlling plant disease according to the present invention can control a plant disease caused by bacteria, particularly bacteria of the *Xanthomonas* genus, in a target plant.

### 3-2. Method

A method for controlling plant disease according to the present invention comprises a contacting step as an essential step.

The "contacting step" is a step of contacting a composition for plant disease control according to the second aspect to the target plant. This step is basically in accordance with "2-3. Method for Application" in the second aspect regarding the composition for plant disease control.

In the present aspect, "contact" refers to contact between a composition for plant disease control and a target plant. More specifically, the contact is between the following: a bacteriolytic agent, i.e., a phage, according to the first aspect as an active component of a composition for plant disease control; and part of a target plant, preferably a site that is infected or to be potentially infected by target bacteria. This step is intended to infect a phage as an active component with target bacteria, whereby the target bacteria are bacteriolyzed. As a result, an effect on controlling the plant disease caused by the target bacteria can be achieved.

The contact may be any of the direct contact and indirect contact. The direct contact in the present aspect means that the composition for plant disease control is brought directly into contact with a predetermined site of the target plant. Specifically, for example, this means that a composition for plant disease control, in liquid form or gel form, is painted, sprayed, scattered, or immersed for part of a target plant. In this case, the part of a plant as a target of contact is mainly a site such as a leaf, flower, fruit, stem, branch, and/or trunk. On the other hand, the indirect contact in the present aspect means that the composition for plant disease control is brought into contact with a predetermined site of the target plant via an intermedium. For example, this means that a composition for plant disease control, in particulate form, is applied into the soil around the root of a target plant. The phage, as an active component, is transported via water or the like in soil, and absorbed by the root.

### 3-3. Effects

A phage, as an active component of a composition obtained by the present invention, can kill target bacteria efficiently, and hence, is useful to prevent and inhibit a disease caused by target bacteria. Additionally, the specific bacteriolytic ability serves as a basis for detecting and identifying target bacteria, thus enabling a disease to be diagnosed. An agent that has been used customarily for bacteria of the *Xanthomonas* genus includes a copper agent or an antibiotic, but such an agent may cause drug poisoning or a disruption of the balance of a bacterial lawn. For example, some strains of *Pseudomonas fluorescens* have proved to have a plant-growth-promoting effect (Haas D., Defago G., Nature Reviews in Microbiology, 2005, 3 (4), 307-19), and a copper agent and an antibiotic have a high possibility of also killing such bacteria having a symbiotic relationship with a plant. On the other hand, since a phage is an organism-derived substance, there is no report of the manifestation of drug poisoning, and since a phage has high specificity, its influence on the balance of a bacterial lawn is extremely limited.

### 4. Method for Identifying Bacteria of the Xanthomonas Genus

### 4-1. Overview

A fourth aspect of the present invention is a method for identifying bacteria of the *Xanthomonas* genus. A method for identification according to the present invention is characterized by utilizing the host specificity of a phage constituting the bacteriolytic agent according to the first aspect to identify bacteria of the *Xanthomonas* genus.

The present invention can determine and identify whether unidentified plant pathogenic bacteria causative of a plant disease are bacteria of the *Xanthomonas* genus.

### 4-2. Method

A method for identification according to the present invention comprises a culturing step, a mixing step, a mixture culturing step, and a determining step as essential steps, and additionally, an isolating step as an optional step. Each step is described below.

### (1) Isolating Step

The "isolating step" is a step of isolating the subject bacteria from the plant tissue affected by a plant disease. This step is an optional step, and may be performed if desired.

The "subject bacteria" refers to plant pathogenic bacteria, the species of which has not been revealed, and that are used for a method for identifying bacteria of the *Xanthomonas* genus in the fourth aspect of the present invention.

The plant tissue may be any site of a plant that has developed a plant disease, and is preferably a site at which the symptom of the plant disease is recognized remarkably. For example, from a peach that has developed a shot hole disease of *Prunus spp.,* a leaf with a disease observed therein may be used.

To isolate subject bacteria from plant tissue taken, a specimen with a disease observed therein may be immersed in a solvent such as water to extract the subject bacteria, and the specimen may be fragmented and crushed upon the extraction. Subsequently, the extraction liquid may be streaked on an agar culture medium, and a single colony may be picked.

### (2) Culturing Step

The "culturing step" is a step of culturing the subject bacteria isolated, and obtaining a culture preparation. Subject bacteria may be cultured by a method known in the art.

The "culture preparation" is obtained by culturing subject bacteria, and may be either liquid or solid.

In this step, subject bacteria are unidentified, and thus, the culture medium to be used in this step is desirably a medium that can culture a wide range of plant pathogenic bacteria. At least a culture medium that can culture bacteria of the *Xanthomonas* genus as target bacteria to be identified in the present invention is used. Such a culture medium may be, for example, a medium comprising one or more components selected from: protein enzymatic decomposition products such as peptone and tryptone; organism-derived extracts such as potato dextrose and yeast extracts; amino acids or salts thereof, such as glutamic acid; saccharides such as glucoses and sucroses; and inorganic salts such as sodium chloride, magnesium chloride, and potassium dihydrogen phosphate. Specific examples of the culture medium and the composition include LB media (tryptone, yeast extract, and sodium chloride), YPG media (yeast extract, peptone, and glucose), PD media (potato dextrose), peptone-supplemented Suwa's media (sucrose, glutamic acid, and peptone), and the like.

The subject bacteria isolated are seeded in the culture medium, and cultured under suitable culture conditions. A culture with stirring under culture conditions, for example, at 20 to 40°C, 20 to 30°C, 22 to 28°C, or 24 to 26°C, yields a culture preparation. The culture time is not limited, and may be any period of time, for example, the culture may be performed until the turbidity at a wavelength of 600 nm reaches approximately 1.0. The present step yields a culture solution of the subject bacteria. Additionally, the culture may be a multi-step culture that is double-step or more. For example, a culture solution obtained by a culture in a solution medium can be supplemented with a soft agar containing liquid medium, poured into a solid medium such as an agar medium, solidified, and subjected to further culture.

### (3) Mixing Step

The "mixing step" is a step of mixing the culture preparation obtained in the culturing step and the bacteriolytic agent according to the first aspect to obtain a mixture.

The "mixture" is a mixture of a culture preparation and a bacteriolytic agent, and may be either liquid or solid.

The mixing method is not particularly limited, as long as it is capable of mixing the culture preparation and the bacteriolytic agent. The bacteriolytic agent according to the first aspect may be in a solid state, or may be suspended in water or a liquid culture medium, and added in a liquid state.

When the culture preparation and the bacteriolytic agent are both liquid, the capacity ratio of the culture preparation to the bacteriolytic agent may be 1:9, 2:8, 3:7, 4:6, 5:5, 6:4, 7:3, 8:2, or 9:1. After the addition, the culture preparation and the bacteriolytic agent may be mixed sufficiently by stirring or the like. On the other hand, when a soft agar containing liquid medium is superposed as described above, the culture preparation is solid. In this case, the bacteriolytic agent may be dropped on a solid culture preparation, such as on the surface of a gel, whereby both are mixed on the solid medium to obtain a mixture.

### (4) Mixture Culturing Step

The "mixture culturing step" is a step of culturing the mixture under predetermined conditions.

Here, in the culture of the mixture, the mixture may also be supplemented with a soft agar containing liquid medium, poured into a solid medium such as an agar medium, solidified, and subjected to further culture.

The basic procedure in this step is in accordance with the culturing step above. In this step, the culture is preferably based on, but not limited to, what is called a plaque assay method so that the bacteriolysis of the subject bacteria by a phage constituting the bacteriolytic agent can be more easily verified in the below-described determining step. For example, after part of the mixture solution is mixed with a soft agar medium having the same composition, and before the soft agar medium is solidified, the resulting mixture may be poured onto an agar medium having the same composition, and spread on the whole culture medium. Then, the mixture may be cultured under the same conditions as in the culturing step.

### (5) Determining Step

The "determining step" is a step of determining that the subject bacteria are bacteria of the *Xanthomonas* genus when the subject bacteria are bacteriolyzed after the culturing step.

Without limitation, for example, when a plaque assay method is used, whether bacteriolysis occurs may be determined according to whether a plaque has been formed. A plaque exists on the soft agar medium, which is spread and solidified on the agar culture medium, after the above-described mixture culturing step, this result indicates that the subject bacteria has been bacteriolyzed by an infection of a phage constituting a bacteriolytic agent of the present invention. Accordingly, the subject bacteria in this case can be determined to be bacteria of the *Xanthomonas* genus. On the other hand, when the subject bacteria proliferate on the whole agar culture medium, but no plaque at all exists, the subject bacteria can be determined not to be bacteria of the *Xanthomonas* genus.

To render the determination more accurate, a negative control and/or a positive control may be prepared simultaneously and used to verify that no plaque is generated in the negative control, and that a plaque is observed in the positive control, wherein the negative control is mixed with a culture medium comprising no bacteriolytic agent in the mixture culturing step, and wherein, for the positive control, bacteria of the *Xanthomonas* genus identified are used from the culturing step rather than subject bacteria.

### 4-3. Effects

A method for identifying bacteria of the *Xanthomonas* genus according to the present invention can identify whether a plant disease is caused by bacteria of the *Xanthomonas* genus.

Additionally, a method for identifying bacteria of the *Xanthomonas* genus according to the present invention can detect whether bacteria of the *Xanthomonas* genus exist in a lesion site of a plant that has developed a plant disease expected to be caused by the bacteria of the *Xanthomonas* genus.

### Examples

### <Example 1: Isolation of Novel Bacteriophage, and Bacteriolytic Ability Thereof (1)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, plant pathogenic bacteria as targets were all obtained from the National Agriculture and Food Research Organization (NARO). Each of the bacteria used in this Example is listed in Table 2, together with the deposition numbering by the National Agriculture and Food Research Organization.

**[Table 2]**

| ID | Species | Source of Isolation | Place of Harvest |
|---|---|---|---|
| MAFF No. 211971 | *Xanthomonas arboricola pv. pruni* | Peach | Yamanashi |
| MAFF No. 311351 | *Xanthomonas arboricola pv. pruni* | Peach | Fukushima |
| MAFF No. 212146 | *Xanthomonas arboricola pv. juglandis* | Walnut | Nagano |
| MAFF No. 301256 | *Xanthomonas campestris pv. vesicatoria* | Tomato | Tokushima |
| MAFF No. 301352 | *Xanthomonas campestris pv. vitians* | Lettuce | Wakayama |
| MAFF No. 311622 | *Xanthomonas arboricola pv. pruni* | Peach | Yamanashi |
| MAFF No. 301078 | *Xanthomonas citri subsp. citri* | Orange | Wakayama |
| NCIMB-ID 10460 | *Pseudomonas fluorescens* | Egg | U.S.A. |

As a control, a *Pseudomonas fluorescens* bacterial strain (NCIMB-ID: 10460) that is reported to have a plant growth promoting action and the like, and is beneficial to the environment was obtained from NCIMB, a laboratory in the United Kingdom National Culture Collection, UKNCC.

For culturing the bacteria of the *Xanthomonas* genus and bacteria of *Pseudomonas fluorescens,* the liquid medium (YPG Broth) prepared by dissolving 1 g of peptone, 1 g of a yeast extract, and 2 g of glucose in 1 L of H₂O and autoclaving, was used. Additionally, as an agar culture medium, agar culture medium (referred to as "YPG Agar" when YPG Broth was used) prepared by adding agar at 15 g per L to the above-described Broth (SW+P Broth or YPG Broth) and autoclaving, was used. Furthermore, to prepare a soft agar medium (Top Agar) to be superposed on the upper layer of the agar culture medium, agarose was added at 5 g per L to the above-described Broth, and autoclaved. The resulting Top Agar was stored at approximately 50°C, and utilized, when necessary.

Each of the above-described bacterial strains sent in a dry powder state was suspended in 0.1 mL of Broth, and then subjected to a streak culture on Agar (YPG Agar) at 25°C, and a single colony was isolated. The isolated colony was inoculated to Broth at 25°C, and subjected to a shaking culture to produce a preculture solution. In main culture, the preculture solution was inoculated to Broth, and cultured at 25°C for 10 to 30 hours until the turbidity (Optical Density at 600 nm) reached approximately 1.0. The culture solution after the culture was directly used as bacteria suspension as it is.

### (2) Isolation and Purification of 1st Phage

A novel phage was isolated from natural dirty water or soil obtained in Japan. The method for isolating a phage was based on a conventional plaque assay method. First, dirty water from a pond, lake, or the like, or dirty water in which soil is suspended in water was filtrated through a 0.45 µm filter to prepare a phage-containing solution. Subsequently, the bacteria suspension and the phage-containing solution were mixed in equal amounts, and left at room temperature for approximately 10 minutes. Next, 0.2 mL of the bacteria/phage mixture solution was added to 3 mL of Top Agar, quickly mixed with a vortex mixer, and then poured on Agar. After the Top Agar was solidified, static culture was performed at 25°C for approximately 12 hours. On the bacterial lawn formed by the culture, a bacteriolytic plaque was formed. Then, a chip with its end cut was used to suck gel from the plaque portion, and a phage having bacteriolytic ability against bacteria of the *Xanthomonas* genus was isolated. Then, the above-described procedure was repeated to purify the phage, using a phage-containing solution comprising a high concentration of the phage isolated was used instead of dirty water.

A total of three kinds of new phages were isolated from natural dirty water and soil, using a method for detecting a bacteriolytic plaque formed on a soft agar medium on which any of the above-described bacterial strains was amplified. One kind of novel phages isolated in Example 1 are referred to as "1st phage".

The isolated phage was suspended in an SM Buffer, passed through a 0.2 µm filter, and collected in the form of a phage-containing solution. This phage-containing solution was mixed with the bacteria suspension under the above-described conditions to isolate a phage again. This procedure was repeated several times to further purify the phage. The composition of the SM Buffer is shown in Table 3.

**[Table 3]**

| SM Buffer | Final | Add to 1L |
|---|---|---|
| NaCl | 0.1 M | 5.8 g |
| Mg₂SO₄·7H₂O | 10 mM | 1 g |
| 1M Tris-HCl pH 8.0 | 50 mM | 50 mL |
| Gelatin | 0.1% | 0.1 g |

### (3) Amplification and Refinement of 1st Phage

The 1st phage isolated and purified was amplified and refined by a plate lysate (PL) method that is an amplifying method using a plaque assay method. In order for many plaques to be formed on Agar, a bacteria/phage mixture solution was prepared, mixed with Top Agar, then spread on YPG Agar, and cultured. Then, 3 mL of SM Buffer was added to the Top Agar having plaques formed thereon, and shaken at 25°C for approximately 30 minutes, and the supernatant was passed through a 0.2 µm filter to collect a collected solution containing a phage.

To refine the 1st phage, 1 g of PEG 6000 (at a final concentration of 10%) and 0.4 g of NaCl (at a final concentration of 4%) were added to and dissolved in 10 mL of the collected solution, and the resulting solution was rotated using a rotator at 4°C overnight. Then, the supernatant was removed by centrifugation under 15,000 × g at 4°C for 60 minutes. The pellets collected were suspended in 0.5 mL of SM Buffer again. Subsequently, 0.5 mL of chloroform was added, and the resulting mixture was stirred vigorously, and left on ice for 6 hours. After centrifugation under 8,000 × g at 4°C for 10 minutes, the upper layer was collected carefully to obtain a refined solution of phage. The concentration of the refined solution of phage is commonly expressed as a titer [PFU/mL] based on the number of plaques (Plaque Forming Unit, PFU) in accordance with a plaque assay method, and serves as one index indicative of bacteriolytic ability. The titer of the refined solution of the 1st phage prepared was determined by a plaque assay method using a solution suitably diluted, and was confirmed to be 10⁸ PFU/mL or more.

### (4) Evaluation of Host Range of 1st Phage

The host range of the 1st phage was evaluated by a spot test method. Only a bacteria suspension in an amount of 0.1 mL was added to and mixed with 3 mL of Top Agar, then poured onto Agar, spread on the whole plate, and solidified. Bacteria suspensions were the bacteria suspensions prepared in (1) above from the bacteria of the *Xanthomonas* genus shown in Table 2 as well as a bacteria suspension prepared as a control from *Pseudomonas fluorescens.* Then, approximately 5 µL of the refined solution of phage was dropped, and subjected to a static culture at 25°C for approximately 12 hours. When the solution became clear in circular shape (approximately 1 cm in diameter) at the site of dropping on the plate having the bacterial lawn formed thereon, it was determined that the phage dropped had bacteriolytic ability against the bacterial strain.

One example of the results is shown in Figures 1 and 2. When the 1st phage exhibited bacteriolytic ability, only the site where the refined solution of phage was dropped became clear in the bacterial lawn formed on the plate. The one kind of 1st phages obtained in the present invention exhibited bacteriolytic ability against the bacterial strains belonging to all three species of bacteria: *Xanthomonas arboricola* (including *pv. pruni* and *pv. juglandis*), *Xanthomonas campestris* (including *pv. vesicatoria* and *pv. vitians)* and *Xanthomonas citri,* which are shown in Table 2. On the other hand, it has also been verified that the phages do not exhibit bacteriolytic ability against *Pseudomonas fluorescens.* There is an example of a report that a phage exhibits the ability against two or more species of bacteria of the *Xanthomonas* genus, but such a pattern as described above has not been reported (Nakayinga R. et al., BMC Microbiology, 2021, 21: 291). This suggests that the 1st phage isolated exhibited bacteriolytic ability against a wide range of bacteria of the *Xanthomonas* genus.

This result suggests the possibility that the 1st phage can be applied to various plant diseases. For example, this suggests the usefulness for control of shot hole disease of *Prunus spp.,* walnut bacterial blight, tomato bacterial blight, lettuce bacterial blight, and orange bacterial canker, which are caused by bacteria of the *Xanthomonas* genus, and is expected to be very valuable in industrial uses.

### (5) Genomic Analysis of 1st Phage

The genomic DNA sequence of the 1st phage was determined and analyzed.

### (i) Preparation and Sequencing of Genomic DNA of 1st Phage

The genome of the 1st phage was extracted using a TURBO DNA-free^{™} kit (Thermo Fisher Scientific Inc.). Host bacteria-derived genomic DNAs, which become contaminants, were removed by a treatment according to the manual attached to the kit. Then, using NucleoSpin^{(R)} Virus (Machery-Nagel & Co. KG), the outer-shell molecules of the phage were decomposed by a Proteinase K treatment according to the attached manual. Via refining the genomic DNA using a silica spin column, a genomic DNA solution of the 1st phage was prepared. Then, the concentration of the genomic DNA was measured using a Qubit dsDNA HS Assay kit (Thermo Fisher Scientific Inc.), and 50 µL of the genomic DNA solution was prepared at a final concentration of 0.2 ng/µL. Subsequently, by a treatment with Nextera XT DNA Library Prep (Illumina, Inc.) and according to the attached manual, the genome of the 1st phage was fragmented, and an adapter sequence was added by PCR. Next, Agilent High Sensitivity DNA Kit (Agilent Technologies, Inc.) was used for electrophoresis with Bioanalyzer (Agilent Technologies, Inc.) to measure the average bp size of the sample, and determine the concentration of the DNA fragments. Lastly, using a Miseq Reagent kit (Illumina, Inc.), a sample for measurement was prepared by a treatment according to the attached manual, and a measurement was made using a next-generation sequencer Miseq (Illumina, Inc.). Utilizing a CLC genomics workbench (Qiagen N.V.), the data obtained were pretreated (for example, trimmed) and then subjected to a de novo assembly to obtain the contig sequence corresponding to the genomic sequence of the phage.

### (ii) Bioinformatics Analysis based on Genomic Sequence Information

On the basis of the nucleotide sequence of the genomic DNA (SEQ ID NO: 1) of the 1st phage obtained in (i) above, and using the NCBI-provided BLAST server (https://blast.ncbi.nlm.nih.gov/Blast.cgi), similar DNA sequences were searched for, and the sequence identities were verified. When known sequences with a high sequence identity were searched for over the range of 90% or more of the full length (Query Cover), a sequence with the highest value of the sequence identity was the genomic DNA sequence of *Pseudomonas* phage vB_Pae_TR (access code: OL802211.1) which had a sequence identity of 99.07% over the range of 93% of the full length of SEQ ID NO: 1.

The sequence identity is a value for the range automatically aligned by an analysis server with respect to the genomic full length of the 1st phage. The range is shown as the value of Query Cover. The sequence identity of 99.07% for vB_Pae_TR is a value calculated over the region limited to 93% of the full length of ΦX-33. Accordingly, the sequence identity to the full length is estimated, although it is difficult to calculate, to be at least a value lower than 99.07% and to correspond to a value equal to or lower than 93%.

It has been revealed that the target bacteria of the phage vB_Pae_TR, which has the genome of the above-described known sequence, are bacteria of *Pseudomonas* genus. Accordingly, for the 1st phage isolated in this Example, it was difficult to identify the target bacteria based on a sequence identity to the genomic sequence of a known phage. Simultaneously, it is highly plausible that a phage having a higher sequence identity to the full length (a phage that exhibits a higher sequence identity over a wider range) than vB_Pae_TR is a phage having the same host range, i.e., diverse bacteria of the *Xanthomonas* genus as host bacteria, as for the 1st phage isolated in this Example.

The above-described results have suggested that the 1st phage is a totally novel phage having a conventionally unreported host range in the *Xanthomonas* genus.

### <Example 2: Isolation of Novel Bacteriophage, and Bacteriolytic Ability Thereof (2)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, the plant pathogenic bacteria as target bacteria were bacteria of the *Xanthomonas* genus, and all the bacterial strains were obtained from the National Agriculture and Food Research Organization (NARO). Each of the bacteria used in Examples 7 and 8 is listed in Table 4, together with the deposition numbering (MAFF No.) by the National Agriculture and Food Research Organization.

**[Table 4]**

| ID | Species | Source of Isolation | Place of Harvest |
|---|---|---|---|
| MAFF No. 311622 | *Xanthomonas arboricola pv. pruni* | Peach | Yamanashi |
| MAFF No. 212146 | *Xanthomonas arboricola pv. juglandis* | Walnut | Nagano |
| MAFF No. 301352 | *Xanthomonas campestris pv. vitians* | Lettuce | Wakayama |
| MAFF No. 106642 | *Xanthomonas campestris pv. campestris* | Fescue | Ibaraki |
| MAFF No. 730097 | *Xanthomonas campestris pv. vesicatoria* | Tomato | Iwate |
| MAFF No. 212147 | *Xanthomonas arboricola pv. juglandis* | Walnut | Nagano |
| MAFF No. 212148 | *Xanthomonas arboricola pv. juglandis* | Walnut | Nagano |
| MAFF No. 212149 | *Xanthomonas arboricola pv. juglandis* | Walnut | Nagano |
| MAFF No. 301256 | *Xanthomonas campestris pv. vesicatoria* | Tomato | Tokushima |
| MAFF No. 301294 | *Xanthomonas campestris pv. vesicatoria* | Tomato | Shizuoka |
| MAFF No. 301257 | *Xanthomonas campestris pv. vesicatoria* | Tomato | Tokushima |
| MAFF No. 301260 | *Xanthomonas campestris pv. vesicatoria* | Pepper | Tokushima |
| MAFF No. 301356 | *Xanthomonas campestris pv. vitians* | Lettuce | Nagano |
| MAFF No. 301354 | *Xanthomonas campestris pv. vitians* | Lettuce | Chiba |
| MAFF No. 301358 | *Xanthomonas campestris pv. vitians* | Lettuce | Fukuoka |
| NCIMB-ID 10460 | *Pseudomonas fluorescens* | Egg | U.S.A. |

Additionally, as a control, a *Pseudomonas fluorescens* bacterial strain (NCIMB-ID: 10460), which is a member of *Pseudomonas* genus, was obtained from NCIMB, a laboratory in the United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 2nd Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil. This novel phage isolated in Example 2 is referred to as a "2nd phage".

The isolated phage was suspended in an SM Buffer, passed through a 0.2 µm filter, and collected in the form of a phage-containing solution. This phage-containing solution was mixed with the bacteria suspension under the above-described conditions to isolate the phage again. This procedure was repeated several times to purify the phage.

### (3) Amplification and Refinement of 2nd Phage

The 2nd phage, isolated and purified, was amplified and refined by the plate lysate (PL) method. Specific method was in accordance with the method described in "(3) Amplification and Refinement of Phage" in Example 1. The titer of the refined solution of the 2nd phage prepared was determined by a plaque assay method using a suitably diluted solution, and was confirmed to be 10⁸ PFU/mL or more.

### (4) Evaluation of Host Range of 2nd Phage

The host range of the 2nd phage was evaluated by the spot test method. The basic operation was in accordance with the method described in "(4) Evaluation of Host Range of 1st Phage" in Example 1.

One example of the results is shown in Figures 3 and 4. When the 2nd phage exhibited bacteriolytic ability, only the site where the refined solution of the phage was dropped became clear in the bacterial lawn formed on the plate. The 2nd phage obtained in the present invention exhibited bacteriolytic ability against all the bacteria of the *Xanthomonas* genus shown in Table 4. On the other hand, the phage did not exhibit bacteriolytic ability against *Pseudomonas fluorescens.* A phage that exhibits bacteriolytic ability against a wide range of bacterial species and the pathovars, such as shown in Table 4, has not been known hitherto. This suggests that the isolated 2nd phage exhibits bacteriolytic ability against a wide range of bacteria of the *Xanthomonas* genus.

There is a high possibility that the 2nd phage, as in the 1st phage, can be applied to various plant diseases. This result has suggested the usefulness for control of, for example, shot hole disease of *Prunus spp.,* walnut bacterial blight, tomato bacterial blight, and lettuce bacterial blight, which are caused by bacteria of the *Xanthomonas* genus.

### (5) Genomic Analysis of 2nd Phage

The genomic DNA sequence of the 2nd phage was determined and analyzed.

### (i) Preparation and Sequencing of Genomic DNA of 2nd Phage

The genome of the 2nd phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the nucleotide sequence of the genomic DNA (SEQ ID NO: 2) of the 2nd phage obtained in (i) above, and using the NCBI-provided BLAST server, similar DNA sequences were searched for, and the sequence identities were verified. As a result of the search, a partial sequence of the genomic DNA of a phage (access code: MT664984.1) targeting bacteria of the *Xanthomonas* genus called Xp12 had a high analogous score and had a sequence identity of 99.03% over the range of 99% of the full length of SEQ ID NO: 2. From this result, the sequence identity to the full length of SEQ ID NO: 2 is estimated to correspond to a value of approximately 98.04%. However, as a bacterial species against which the phage Xp12 exhibits bacteriolytic ability, only *Xanthomonas oryzae* is known (Nakayinga R. et al., BMC Microbiology, 2021, 21: 291).

The full length of the genomic DNA sequence of Xp12 is 63,783 nucleotides, whereas the full length of the genomic DNA sequence of the 2nd phage isolated in this Example was 35,321 nucleotides, which was about half the full length of Xp12. As a result, when the sequence identity was calculated using GENETYX-NGS packaged in genetic information processing software GENETYX (https://www.genetyx.co.jp/) with reference to the genomic DNA sequence of Xp12 contrary to the above description, the nucleotide sequence (SEQ ID NO: 2) of the genomic DNA of the 2nd phage had a sequence identity of 98.34% over the range of 54% of the full length of the genomic DNA sequence of Xp12. From this result, the sequence identity between the full lengths of the genomic DNA sequences of these phages compared was estimated to be approximately 53.10%. Thus, the genomic DNA sequence of the 2nd phage obtained in this Example merely has a sequence partially homologous to the genomic DNA sequence of Xp12 and is not highly analogous thereto as a whole.

Differences between these genomic DNA sequences were further analyzed by comparing the genomic DNA sequences of Xp12 and the phage of the present invention using MUMmer packaged in genetic information processing software GENETYX (https://www.genetyx.co.jp/). As a result, a sequence homologous to a region from the 12,894th to the 41,320th of the genomic DNA sequence of Xp12 was not detected in the nucleotide sequence of SEQ ID NO: 2, revealing that the region was deleted in the genomic DNA sequence of the 2nd phage. The region contained a gene (access code: QNN97189.1) estimated as a tail fiber gene from the identity of the sequence, a tail fiber gene (access code: QNN97196.1), and an endolysin gene (access code: QNN97201.1). These genes are genes deeply involved in the process of infection of hosts. This has also suggested that the 2nd phage and Xp12 differ largely in morphology and properties, and this difference leads to the difference in host range. In addition, although there existed phages having a nucleotide sequence having a relatively high analogous score to the nucleotide sequence of SEQ ID NO: 2, all the phages had a full length of more than 60,000 nucleotides, as in the genomic DNA sequence of Xp12, and had a high analogous score to the full length of the genomic DNA sequence of Xp12.

The search range was further expanded, and up to a sequence having a sequence identity of 80% or more was searched for over the range of 80% or more of the full length of the genomic DNA sequence of the 2nd phage (Query Cover). However, a known sequence having a high analogous score to the genomic DNA sequence of the 2nd phage and having a full length of 60,000 or less nucleotides in the genomic DNA sequence was not detected in the NCBI-provided BLAST server.

The above-described results have suggested that the 2nd phage is a totally novel phage having properties changed by partial deletion of the genomic DNA.

### <Example 3: Isolation of Novel Bacteriophage, and Bacteriolytic Ability Thereof (3)>

### (Purpose)

The purpose is to isolate a novel bacteriophage having bacteriolytic ability against pathogenic bacteria of a plant disease, and verify the bacteriolytic ability against the plant pathogenic bacteria.

### (Methods and Results)

### (1) Obtainment and Culture of Plant Pathogenic Bacteria

In this Example, the plant pathogenic bacteria were bacteria of the *Xanthomonas* genus, and all the bacterial strains were obtained from the National Agriculture and Food Research Organization (NARO). Each of the bacteria used in this Example is listed in Table 5, together with the deposition numbering (MAFF No.) by the National Agriculture and Food Research Organization.

**[Table 5]**

| ID | Species | Source of Isolation | Place of Harvest |
|---|---|---|---|
| MAFF No. 212146 | *Xanthomonas arboricola pv. juglandis* | Walnut | Nagano |
| MAFF No. 212147 | *Xanthomonas arboricola pv. juglandis* | Walnut | Nagano |
| MAFF No. 301294 | *Xanthomonas campestris pv. vesicatoria* | Tomato | Shizuoka |
| MAFF No. 301257 | *Xanthomonas campestris pv. vesicatoria* | Tomato | Tokushima |
| MAFF No. 106692 | *Xanthomonas campestris pv. campestris* | Cabbage | Ibaraki |
| MAFF No. 106765 | *Xanthomonas campestris pv. campestris* | Broccoli | Ibaraki |
| MAFF No. 301359 | *Xanthomonas campestris pv. vitians* | Lettuce | Gunma |
| NCIMB-ID 10460 | *Pseudomonas fluorescens* | Egg | U.S.A. |

As a control, a *Pseudomonas fluorescens* bacterial strain (NCIMB-ID: 10460), which is member of *Pseudomonas* genus, was obtained from NCIMB, a laboratory in the United Kingdom National Culture Collection, UKNCC.

Bacteria of the *Xanthomonas* genus and *Pseudomonas fluorescens* were cultured in accordance with the method described in "(1) Obtainment and Culture of Plant Pathogenic Bacteria" in Example 1.

### (2) Isolation and Purification of 3rd Phage

A novel phage was isolated from natural dirty water or soil obtained in Japan. A method for isolation and a method for purification were in accordance with the methods described in "(2) Isolation and Purification of 1st Phage" in Example 1. Consequently, one kind of new phage was isolated from natural dirty water and soil. This novel phage isolated in Example 3 is referred to as a "3rd phage".

The isolated phage was suspended in an SM Buffer, passed through a 0.2 µm filter, and collected in the form of a phage-containing solution. This phage-containing solution was mixed with the bacteria suspension under the above-described conditions to isolate the phage again. This procedure was repeated several times to purify the phage.

### (3) Amplification and Refinement of 3rd Phage

The 3rd phage, isolated and purified, was amplified and refined by the plate lysate (PL) method. Specific method was in accordance with the method described in "(3) Amplification and Refinement of Phage" in Example 1. The titer of the refined solution of the 3rd phage prepared was determined by a plaque assay method using a suitably diluted solution, and was confirmed to be 10⁸ PFU/mL or more.

### (4) Evaluation of Host Range of 3rd Phage

The host range of the 3rd phage was evaluated by the spot test method. The basic operation was in accordance with the method described in "(4) Evaluation of Host Range of 1st Phage" in Example 1.

One example of the results is shown in Figure 5. When the 3rd phage exhibited bacteriolytic ability, only the site where the refined solution of the phage was dropped became clear in the bacterial lawn formed on the plate. The 3rd phage obtained in the present invention exhibited bacteriolytic ability against all the bacteria of the *Xanthomonas* genus shown in Table 5. On the other hand, the phage did not exhibit bacteriolytic ability against *Pseudomonas fluorescens.* A phage that exhibits bacteriolytic ability against a wide range of bacterial species and the pathovars, such as shown in Table 5, has not been known hitherto. This suggests that the 3rd phage isolated exhibited bacteriolytic ability against a wide range of bacteria of the *Xanthomonas* genus.

This result suggested that the third phage is useful for controlling walnut bacterial blight, tomato bacterial blight, lettuce bacterial blight, black rot disease of broccoli, and the like, which are caused by bacteria of the *Xanthomonas* genus.

### (5) Genomic Analysis of 3rd Phage

The genomic DNA sequence of the 3rd phage was determined and analyzed.

### (i) Preparation and Sequencing of Genomic DNA of 3rd Phage

The genome of the 3rd phage was extracted using a TURBO DNA-free^{™} kit (Thermo Fisher Scientific Inc.). The basic operation was in accordance with the method described in "(5) Genomic Analysis of 1st Phage, (i) Preparation and Sequencing of Genomic DNA of 1st Phage" in Example 1.

### (ii) Bioinformatics Analysis based on Genomic Sequence Information

On the basis of the nucleotide sequence of the genomic DNA (SEQ ID NO: 3) of the 3rd phage obtained in (i) above, and using the NCBI-provided BLAST server, similar DNA sequences were searched for, and the sequence identities were verified. As a result of the search, the genomic DNA sequence of a phage (access code: OK490494.1) targeting bacteria of the *Stenotrophomonas* genus called vB_SmaS_P15 had the highest analogous score and had a sequence identity of 85.20% (Query Cover: a sequence identity of 96.82% over the range of 88% of the full length of SEQ ID NO: 3. From this result, the sequence identity to the full length is estimated to correspond to a value of approximately 85.20%. Likewise, all of a known phage having the second highest genomic DNA sequence identity (phage name: BUCT598, access code: MW831865.1, the sequence identity to the full length: approximately 85.18%) and known phages having the third highest genomic DNA sequence identity (phage name: BUCT703 and BUCT700, access code: OM735688.1 and OM735686.1, respectively, the sequence identity to the full length: approximately 56.7% for both) targeted bacteria of the *Stenotrophomonas* genus.

This result suggested that the 3rd phage is a totally novel phage with bacteria of the *Xanthomonas* genus as hosts.

### <Example 4: Bacteriolytic Ability of a Combination of Bacteriophages Obtained (1)>

### (Purpose)

The purpose is to examine how novel bacteriophages, shown to have bacteriolytic ability against the pathogenic bacteria of a plant disease when used alone, have an effect on the plant disease when used in combination.

### (Method)

### (1) Spot Test Method

A spot test was performed in accordance with the method described in "(4) Evaluation of Host Range of 1st Phage" in Example 1. In this Example, a bacterial strain of *Xanthomonas cucurbitae* (MAFF No. 673005) and *Xanthomonas campestris pv. vitians* (MAFF No. 301352), against which all the phages tested were confirmed to have bacteriolytic ability, was used.

As a refined solution of phage to be dropped, a solution mixture obtained by mixing equal amounts of the refined solutions of phage prepared in each Example was used. The solution mixture was prepared by being suitably diluted so that the total titer could be the same as when the solution was used alone. The phages used in this Example are as below.

In this Example, the phage having the genomic DNA sequence of SEQ ID NO: 4 (b in Figure 6B), the phage having the genomic DNA sequence of SEQ ID NO: 5 (c in Figure 6B), the phage having the genomic DNA sequence of SEQ ID NO: 6 (d in Figure 6B), the phage having the genomic DNA sequence of SEQ ID NO: 7 (e in Figure 6B), the 2nd phage having the genomic DNA sequence of SEQ ID NO: 2 (f in Figure 6B), the 3rd phage having the genomic DNA sequence of SEQ ID NO: 3 (g in Figure 6B), and the phage having the genomic DNA sequence of SEQ ID NO: 8 (h in Figure 6B) were used in combination with the 1st phage having the genomic DNA sequence of SEQ ID NO: 1 (a in Figure 6B).

### (Results)

One example of the results is shown in Figure 6. When a combination of the phages exhibited bacteriolytic ability, only the site where the refined solution of the phage was dropped became clear in the bacterial lawn formed on the plate. Using any of the combinations of two kinds tested has been revealed to exhibit bacteriolytic ability at least in the same manner as using each phage alone (Figure 6).

The results have verified that phages of the present invention are useful when used in combination as well as when used alone.

### <Example 5: Bacteriolytic Ability of a Combination of Bacteriophages Obtained (2)>

### (Purpose)

The purpose is to examine how novel bacteriophages, shown to have bacteriolytic ability against the pathogenic bacteria of a plant disease when used alone, have an effect on the plant disease when used in combination.

### (Method)

### (1) Spot Test Method

A spot test was performed in accordance with the method described in "(4) Evaluation of Host Range of 1st Phage" in Example 1. In this Example, bacterial strains of *Xanthomonas cucurbitae* (MAFF No. 673005) and *Xanthomonas campestris pv. vitians* (MAFF No. 301352), against which all the phages tested were confirmed to have bacteriolytic ability, was used.

As a refined solution of phage to be dropped, a solution mixture obtained by mixing equal amounts of the refined solutions of phage prepared in each Example was used. The solution mixture was prepared by being suitably diluted so that the total titer could be the same as when the solution was used alone. The phages used in this Example are as below.

In this Example, the phage having the genomic DNA sequence of SEQ ID NO: 4 (b in Figure 7B), the phage having the genomic DNA sequence of SEQ ID NO: 5 (c in Figure 7B), the phage having the genomic DNA sequence of SEQ ID NO: 6 (d in Figure 7B), the phage having the genomic DNA sequence of SEQ ID NO: 7 (e in Figure 7B), the 1st phage having the genomic DNA sequence of SEQ ID NO: 1 (f in Figure 7B), the 3rd phage having the genomic DNA sequence of SEQ ID NO: 3 (g in Figure 7B), and the phage having the genomic DNA sequence of SEQ ID NO: 8 (h in Figure 7B) were used in combination with the 2nd phage having the genomic DNA sequence of SEQ ID NO: 2 (a in Figure 7B).

### (Results)

One example of the results is shown in Figure 7. When a combination of the phages exhibited bacteriolytic ability, only the site where the refined solution of the phage was dropped became clear in the bacterial lawn formed on the plate. Using any of the combinations of two kinds tested has been revealed to exhibit bacteriolytic ability at least in the same manner as using each phage alone (Figure 7).

The results have verified that phages of the present invention are useful when used in combination as well as when used alone.

### <Example 6: Bacteriolytic Ability of a Combination of Bacteriophages Obtained (3)>

### (Purpose)

The purpose is to examine how novel bacteriophages, shown to have bacteriolytic ability against the pathogenic bacteria of a plant disease when used alone, have an effect on the plant disease when used in combination.

### (Method)

### (1) Spot Test Method

A spot test was performed in accordance with the method described in "(4) Evaluation of Host Range of 1st Phage" in Example 1. In this Example, bacterial strains of *Xanthomonas cucurbitae* (MAFF No. 673005), *Xanthomonas campestris pv. vesicatoria* (MAFF No. 301257), and *Xanthomonas campestris pv. campestris* (MAFF No. 106765), against which all the phages tested were confirmed to have bacteriolytic ability, was used.

As a refined solution of phage to be dropped, a solution mixture obtained by mixing equal amounts of the refined solutions of phage prepared in each Example was used. The solution mixture was prepared by being suitably diluted so that the total titer could be the same as when the solution was used alone. The phages used in this Example are as below.

In this Example, the phage having the genomic DNA sequence of SEQ ID NO: 4 (b in Figure 8B), the phage having the genomic DNA sequence of SEQ ID NO: 5 (c in Figure 8B), the phage having the genomic DNA sequence of SEQ ID NO: 6 (d in Figure 8B), the phage having the genomic DNA sequence of SEQ ID NO: 7 (e in Figure 8B), the 1st phage having the genomic DNA sequence of SEQ ID NO: 1 (f in Figure 8B), the 2nd phage having the genomic DNA sequence of SEQ ID NO: 2 (g in Figure 8B), and the phage having the genomic DNA sequence of SEQ ID NO: 8 (h in Figure 8B) were used in combination with the 3rd phage having the genomic DNA sequence of SEQ ID NO: 3 (a in Figure 8B).

### (Results)

One example of the results is shown in Figure 8. When a combination of the phages exhibited bacteriolytic ability, only the site where the refined solution of the phage was dropped became clear in the bacterial lawn formed on the plate. Using any of the combinations of two kinds tested has been revealed to exhibit bacteriolytic ability at least in the same manner as using each phage alone (Figure 8).

The results have verified that phages of the present invention are useful when used in combination as well as when used alone.

### <Example 7: Disease Control Effect Test on Tomato Bacterial Blight>

### (Purpose)

The purpose is to examine how isolated novel bacteriophages with bacteriolytic ability against the pathogenic bacteria of a plant disease have an effect on the plant disease when applied to a plant.

### (Method)

### (1) Preparation of Spreading Solution of Phage

A spreading solution of phage used in this test was prepared in accordance with the following procedure.

First, a bacterial cell culture solution of host bacteria for amplifying phages was prepared in accordance with the following procedure. As the host, a bacterial strain of *Xanthomonas campestris pv. vesicatoria* (MAFF No. 301256), against which all the phages tested were confirmed to have bacteriolytic ability, was used. The bacterial cell of this strain was inoculated into YPG Broth and incubated overnight with a shaker set at 25°C. After the incubation, OD₆₀₀ (a turbidity at a wavelength of 600 nm) was measured, and a bacterial cell culture solution, the turbidity of which became approximately 1.0, was used below.

The bacterial cell culture solution prepared and a refined solution of phage containing one kind of phage (having a titer of approximately 10⁸ PFU/mL) prepared in Example 1 were mixed in equal amounts. The resulting mixture solution was inoculated into a 100-fold amount of YPG culture solution and incubated for approximately 8 to 12 hours with a shaker set at 25°C. The resulting culture solution was collected as a crude solution of phage. To the crude solution collected, a 1/10 amount of chloroform was added, stirred vigorously, and then centrifuged at 8,000 × g at 20°C for 5 minutes. The supernatant was then collected. The collected supernatant was passed through a 0.2 µm filter, and the resulting filtrate was used as a refined solution of phage.

As a spreading solution of phage comprising one kind of phage, a refined solution of phage diluted with sterile tap water so as to have a titer of approximately 10⁹ PFU/mL was used.

### (2) Preparation of Spreading Solution of Bacteria

To prepare a spreading solution of bacteria used to infect a plant in this test, the bacterial cell culture solution prepared in (1) was used. A bacterial cell culture solution diluted approximately 10,000-fold with sterile tap water was applied to a YPG Agar plate, and incubated for approximately 1 to 3 days in an incubator set at 25°C. A bacterial cell suspension, in which the colony on the YPG Agar plate was collected by suspending in sterile tap water, was diluted with sterile tap water so that a final OD₆₀₀ was approximately 0.5, and the resulting solution was used as a spreading solution of bacteria.

### (3) Plant Specimen

Commercially available seeds of tomatoes were seeded and grown in a greenhouse. A seedling grown to have 50 or more leaves was used as a specimen for evaluation.

### (4) Application and Infection of Phage

With the phage-applied group, foliar application of the spreading solution of phage to each specimen was performed twice each, twice at intervals of 2 to 3 days, before and after the bacterial infection, and was followed by foliar application of the spreading solution of bacteria 2 to 3 days later, whereby the specimen was infected with the bacteria. Over an approximately 2-day period after the infection treatment, the specimen infected with the bacteria was left in a vinyl house with a high humidity being maintained. Then, another foliar application of the spreading solution of phage, twice with an interval of two to three days, was performed again. For a nontreated group, the same procedure as described above was performed except that sterile tap water was used instead of a refined solution of phage.

### (5) Measurement of Incidence Rate

16 days after the infection treatment, the nontreated group and each phage-applied group were examined for the incidence rate.

A leaf, on the face of which a characteristic of tomato bacterial blight was developed, was judged as a diseased leaf. The ratio of the number of diseased leaves out of the total number of all the leaves was calculated as an incidence rate. The relative incidence rate of the phage-applied group was calculated as a relative value, with the incidence rate of the nontreated group assumed to be 100%.

### (Results)

The results are shown in Figure 9. The incidence rate of the nontreated group was approximately 25%. On the other hand, the relative incidence rate, with the incidence rate of the nontreated group assumed to be 100%, was approximately 51.1% when the spreading solution of phage comprising the 1st phage was applied.

The results have revealed that, even when applied to an actual plant, a phage of the present invention can effectively control the plant away from a disease.

In the phage-applied group, there was no significant adverse effect found on any plant that could be attributed to the phage, such as drug poisoning found in a leaf. This has revealed that applying a phage of the present invention is an effective disease control means with a smaller side effect.

### <Example 8: Disease Control Effect Test on Black Rot Disease of Broccoli>

### (Purpose)

The purpose is to examine how isolated novel bacteriophages with bacteriolytic ability against the pathogenic bacteria of a plant disease have an effect on the plant disease when applied to a plant.

### (Method)

### (1) Preparation of Spreading Solution of Phage

A spreading solution of phage was prepared in accordance with Example 7, except that the bacteria used as a bacterial cell was *Xanthomonas campestris pv. campestris* (MAFF No. 106765), and that the below-described phages were used.

For a spreading solution of phage comprising a plurality of kinds of phages, refined solutions of phage adjusted so as to each have a titer at the same level (order), mixed in equal amounts, and diluted with sterile tap water so as to have a titer of approximately 10⁹ PFU/mL were used.

In this Example, the 3rd phage having the genomic DNA sequence of SEQ ID NO: 3 (a in Figure 10) and the phage having the genomic DNA sequence of SEQ ID NO: 9 (b in Figure 10), or additionally the phage having the genomic DNA sequence of SEQ ID NO: 8 (c in Figure 10) and the phage having the genomic DNA sequence of SEQ ID NO: 10 (d in Figure 10), or further additionally the phage having the genomic DNA sequence of SEQ ID NO: 5 (e in Figure 10), the phage having the genomic DNA sequence of SEQ ID NO: 6 (f in Figure 10), the phage having the genomic DNA sequence of SEQ ID NO: 7 (g in Figure 10), and the phage having the genomic DNA sequence of SEQ ID NO: 11 (h in Figure 10) were used in combination.

### (2) Preparation of Spreading Solution of Bacteria

A spreading solution of phage was prepared in accordance with Example 7, except that the bacterial cells were the above-described bacteria.

### (3) Plant Specimen

Commercially available seeds of broccoli were seeded, and grown in a greenhouse. A seedling grown to have five or more leaves was used as a specimen for evaluation.

### (4) Application and Infection of Phage

This was performed in accordance with Example 7.

### (5) Measurement of Incidence Rate

This was performed the same as in Example 7, except that an evaluation was made 16 days after the infection treatment.

### (Results)

The results are shown in Figure 10. The incidence rate of the nontreated group was approximately 52%. On the other hand, the relative incidence rate, with the incidence rate of the nontreated group assumed to be 100%, was approximately 56% when the spreading solution of phage comprising only one kind of the 3rd phage was applied ("a" in Figure 10).

Furthermore, when the spreading solution of phage comprising two kinds or more of phages was applied, the incidence rate was further decreased to approximately 45.7% on average. Although the relative incidence rate was approximately 60% in the case of combining the 3rd phage with one type of phage (a total of two types) ("a/b" in Figure 10), an increase in the number of types to be combined tended to enhance the disease control effect and decrease the relative incidence rate as a whole. Specifically, the relative incidence rate was approximately 43.5% in the case of combining a total of four types ("a/b/c/d" in Figure 10) and approximately 33.5% in the case of combining a total of eight types ("a/b/c/d/e/f/g/h" in Figure 10).

The results have revealed that, even when applied to an actual plant, a phage of the present invention can effectively control the plant away from a disease, regardless of the kind of plant itself. Additionally, applying the phages of the present invention in combination has been revealed to afford a higher effect.

In the applied-phage group, there was no significant adverse effect found on any plant that could be attributed to the phage, such as drug poisoning found in a leaf. This has revealed that applying a phage of the present invention is an effective disease control means with a smaller side effect.

All publications, patents, and patent applications cited herein are incorporated herein by reference in their entirety.

## Claims

1. A bacteriolytic agent comprising a bacteriophage having a genomic DNA sequence comprising the nucleotide sequence of any of the following (1a) to (1c):
(1a) the nucleotide sequence of SEQ ID NO: 1;
(1b) the nucleotide sequence having addition, deletion, and/or substitution of one or a plurality of nucleotides in the nucleotide sequence of SEQ ID NO: 1; or
(1c) a nucleotide sequence having a sequence identity of 95% or more to the nucleotide sequence of SEQ ID NO: 1.

2. A bacteriolytic agent comprising a bacteriophage having a genomic DNA sequence comprising the nucleotide sequence of any one of the following (3a) to (3c):
(3a) the nucleotide sequence of SEQ ID NO: 3;
(3b) the nucleotide sequence having addition, deletion, and/or substitution of one or a plurality of nucleotides in the nucleotide sequence of SEQ ID NO: 3; or
(3c) a nucleotide sequence having a sequence identity of 90% or more to the nucleotide sequence of SEQ ID NO: 3.

3. A bacteriolytic agent comprising a bacteriophage having a genomic DNA sequence comprising the nucleotide sequence of any one of the following (2a) to (2c) with a full length of 40,000 or less nucleotides:
(2a) the nucleotide sequence of SEQ ID NO: 2;
(2b) the nucleotide sequence having addition, deletion, and/or substitution of one or a plurality of nucleotides in the nucleotide sequence of SEQ ID NO: 2; or
(2c) a nucleotide sequence having a sequence identity of 90% or more to the nucleotide sequence of SEQ ID NO: 2.

4. The bacteriolytic agent according to any one of claims 1 to 3, wherein the bacteriolytic agent exhibits bacteriolytic ability against bacteria of the *Xanthomonas* genus.

5. A composition comprising the bacteriolytic agent according to any one of claims 1 to 4 as an active component.

6. A composition for plant disease control, comprising the composition according to claim 5.

7. The composition for plant disease control according to claim 6, wherein the plant disease is a plant disease caused by bacteria of the *Xanthomonas* genus.

8. The composition for plant disease control according to claim 6, comprising another bacteriophage having bacteriolytic ability against the bacteria of the *Xanthomonas* genus.

9. A method for controlling plant disease, comprising a contacting step of contacting the composition for plant disease control according to claim 6 with a target plant.

10. A method for identifying bacteria of the *Xanthomonas* genus, comprising:
a culturing step of culturing subject bacteria isolated from a plant tissue affected by a plant disease, to obtain a culture preparation;
a mixing step of mixing the culture preparation and the bacteriolytic agent according to claim 4, to obtain a mixture;
a mixture culturing step of culturing the mixture under predetermined conditions; and
a determining step of determining that the subject bacteria are bacteria of the *Xanthomonas* genus, when the subject bacteria are bacteriolyzed after the mixture culturing step.

11. The method according to claim 10, wherein, in the mixture culturing step, the mixture further comprises a soft agar containing liquid medium, and the mixture is cultured on a solid medium.

12. The method according to claim 10, wherein, in the culturing step, the culture preparation comprises a soft agar containing liquid medium, and the culture preparation is cultured on a solid medium.

13. The method according to claim 10, further comprising, before the culturing step, an isolating step of isolating subject bacteria from a plant tissue affected by a plant disease.
